# EUROPÄISCHE PATENTSCHRIFT

(11) **EP 1 715 944 B1**
(45) Veröffentlichungstag und Bekanntmachung des Hinweises auf die Patenterteilung: **11.06.2008**
(21) Anmeldenummer: 05701399.7
(22) Anmeldetag: 11.02.2005
(51) Int. Cl.: B01F 17/00

(54) **MISCHUNG, UMFASSEND EIN TENSID UND EIN COTENSID**
MIXTURE COMPRISING A DETERGENT AND A CO-DETERGENT
MELANGE CONTENANT UN TENSIOACTIF ET UN CO-TENSIOACTIF

(30) Priorität: 13.02.2004 DE 102004007473
(43) Veröffentlichungstag der Anmeldung: 02.11.2006
(73) Patentinhaber: BASF SE, 67056 Ludwigshafen (DE)
(72) Erfinder: MOCK-KNOBLAUCH, Cordula, 67063 Ludwigshafen (DE); WAGNER, Norbert, 67112 Mutterstadt (DE); OETTER, Günter, 67227 Frankenthal (DE); VÖLKEL, Ludwig, 67117 Limburgerhof (DE); PETROVIC, Susanne, 69214 Eppelheim (DE); LANGE, Arno, 67098 Bad Dürkheim (DE); MIJOLOVIC, Darijo, 68309 Mannheim (DE); HÜFFER, Stephan, 67063 Ludwigshafen (DE)
(74) Vertreter: Isenbruck, Günter
(86) Internationale Anmeldenummer: PCT/EP2005/001370
(87) Internationale Veröffentlichungsnummer: WO 2005/077513

(56) Entgegenhaltungen:
- DE-A- 10 118 480
- DE-A- 10 215 108

## Beschreibung

Die Erfindung betrifft ein Mischung, umfassend ein Tensid und ein Cotensid, eine Mikroemulsion, enthaltend ein Tensid und ein Cotensid, die Verwendung einer Mischung oder einer Mikroemulsion sowie Wasch-, Reinigungs-, Netz-, Beschichtungs-, Klebe-, Lederentfettungs-, Feuchthalte- oder Textilbehandlungsmittel oder pharmazeutische, Pflanzenschutz- oder kosmetische Formulierung, insbesondere Sonnenschutz-Hautpflege- oder Haarstylingmittel, Duschgele, Shampoos, Badezusätze oder Duftöle.

Tenside sind Substanzen, die die Grenzflächenspannung zwischen miteinander nicht mischbaren flüssigen Phasen, einer polaren Phase, häufig Wasser und einer unpolaren, organischen Phase, herabsetzen und somit ihre gegenseitige Löslichkeit erhöhen. Tenside verfügen über einen charakteristischen Aufbau und weisen mindestens eine hydrophile und eine hydrophobe Struktureinheit auf. Dieser Aufbau wird auch als amphiphil bezeichnet.

Tenside sind ökologisch besonders relevante Stoffe, deren Umweltverträglichkeit sichergestellt werden muss. Neben einer guten Abbaubarkeit von Tensidrückständen in Abwässern ist es daher besonders wichtig, die eingesetzten Tensidmengen möglichst ohne Beeinträchtigung ihrer Wirksamkeit herabzusetzen, das heißt die Effizienz der Tenside zu steigern. Als Tensideffizienz wird dabei üblicherweise die Menge an Tensid bezeichnet, die benötigt wird, um einen bestimmten Effekt zu erzielen, zum Beispiel um den Anteil unpolarer Phase in der polaren Phase bzw. umgekehrt zu solubilisieren oder um die Oberflächenspannung bei möglichst niedriger Konzentration möglichst stark zu verringern.

Übliche konventionelle Emulsionen können Öl- und Wasserphase in sehr unterschiedlichen Volumenanteilen enthalten. Sie haben eine kontinuierliche und eine disperse Phase, die als sehr kleine, durch Belegung mit Tensiden stabilisierte Kügelchen in der kontinuierlichen Phase vorliegt. Je nach der Natur der kontinuierlichen Phase spricht man von Öl-in-Wasser- oder Wasser-in-Öl-Emulsionen. Diese Emulsionen sind im Idealfall kinetisch stabil, d. h. sie bleiben auch längere Zeit, aber nicht unbegrenzt, erhalten. Insbesondere bei Temperaturschwankungen können sie zur Phasentrennung durch Absitzen, Aufrahmen, Verdicken oder Flocken neigen.

Sogenannte Mikroemulsionen sind thermodynamisch stabile, fluide, optisch klare Formulierungen zweier nicht mischbarer Flüssigkeiten, wie Öl und Wasser. Mikroemulsionen entstehen, wenn ein Tensid, oder häufiger eine Mischung aus einem Tensid und einem Cotensid die Grenzflächenspannung Öl/Wasser auf extrem niedrige Werte häufig im Bereich -10⁻³ bis 10⁻⁹, bevorzugt 10⁻⁴ bis 10⁻⁶ N/m absenkt, so dass durch die thermische Bewegung homogen die beiden unlöslichen Phasen allein dispergiert bleiben. Mikroemulsionen weisen häufig bikontinuierliche Strukturen auf mit Gleichgewichtsbereichen, sogenannten Sub-Phasen in der Größenordnung von 100 bis 1000 Angström (vgl. Advanced Materials, 2000, 12, Nr. 23, Seiten 1751 ff.).

Bikontinuierliche Mikroemulsionen enthalten zwei Phasen, eine Wasser- und eine Ölphase, in Form von ausgedehnten nebeneinanderliegenden und ineinander verschlungenen Domänen, an deren Grenzfläche stabilisierende grenzflächenaktive Tenside in einer monomolekularen Schicht angereichert sind. Bikontinuierliche Mikroemulsionen bilden sich sehr leicht, in der Regel wegen der sehr niedrigen Grenzflächenspannung spontan, wenn die Einzelkomponenten, Wasser, Öl und ein geeignetes grenzflächenaktives System, vermischt werden. Da die Domänen in mindestens einer Dimension nur sehr geringe Ausdehnungen in der Größenordnung von Nanometern haben, erscheinen die Mikroemulsionen visuell transparent und sind je nach dem eingesetzten grenzflächenaktiven System in einem bestimmten Temperaturbereich thermodynamisch, das heißt zeitlich unbegrenzt, stabil.

Bikontinuierliche Mikroemulsionen sind zum Beispiel in dem Artikel "Mikroemulsionen - eine wissenschaftliche und anwendungstechnische Fundgrube?" von H.-F. Eicke in SÖFW-Journal 118 (1992), Seiten 311 bis 314, beschrieben.

Zum Erreichen der erforderlichen niedrigen Grenzflächenspannung an den Phasengrenzen enthalten die Mikroemulsionen spezielle Amphiphile, das heißt grenzflächenaktive Mittel, und in ihrer wässrigen Phase häufig gelöste Elektrolyte und gegebenenfalls weitere Hilfsstoffe. Elektrolyte werden vor allem dann zugesetzt, wenn die Amphiphile zum Teil oder ausschließlich ionische Tenside sind.

Aus DE-A 198 39 054 ist es bekannt, die Effizienz von Tensiden durch Zugabe von Additiven zu steigern, wobei als Additive AB-Blockcopolymere mit einem wasserlöslichen Block A und einem wasserunlöslichen Block B eingesetzt werden. Die Blöcke A und B können dabei Molekulargewichte zwischen 500 und 60.000 g/mol annehmen. Als Block A wird bevorzugt ein Polyethylenoxid-Block eingesetzt, allgemein jedoch alle wasserlöslichen Blöcke, die in Verbindung mit Block B ein Amphiphil bilden. Für den Block B werden Polymerisate eines einzigen Monomers oder eines Monomerengemisches beschrieben.

Die beschriebenen Blockcopolymere haben jedoch insbesondere den Nachteil, dass sie nach Herstellungsverfahren erhältlich sind, die für den Labormaßstab, nicht jedoch für den großtechnischen Einsatz geeignet sind. Die genannte Druckschrift verweist zum Herstellungsverfahren auf die DE-A 196 34 477, worin die Polymerisation unter Verwendung von Alkalimetallorganylen beschrieben wird, das heißt eine für den großtechnischen Einsatz ungeeignete Herstellungsmethode.

Dokument DE 101 18 480 beschreibt Mischungen umfassend ein Tensid und ein AB-Bockcopolymerisat als Additiv sowie Anwendungsgebiete hierfür.

Dokument DE-A 102 15 108 beschreibt Polyisobutenamine, deren Polyisobutenreste eine Polydispersität von kleiner oder gleich 1 oder 4 aufweisen, ein Verfahren zur Herstellung sowie die Verwendung der Polyisobutenamine als Kraftstoffadditive. Die beschriebenen Polyisobutenamine zeichnen sich durch eine verbesserte Detergenswirkung als auch durch verbesserte Viskositätseigenschaften, insbesondere bei tiefer Temperatur, aus. Aufgrund dieser vorteilhaften Eigenschaften können sie mit deutlich geringeren Mengen an Hilfsstoffen, wie Trägerölen, eingesetzt werden, wobei eine gute bis sehr gute Wirksamkeit bei deutlich reduziertem Gesamtanteil an Additiven erreicht wird.

Es war demgegenüber Aufgabe der Erfindung, Substanzen zur Verfügung zu stellen, die als Cotenside zur Steigerung der Effizienz von Tensiden in Emulsionen, insbesondere in Mikroemulsionen, einsetzbar sind, und die in wirtschaftlich vorteilhafter Weise, auf der Basis von großtechnischen Ausgangssubstanzen sowie auf großtechnisch realisierbaren Umsetzungswegen erhalten werden können. Insbesondere soll eine Steigerung der Effizienz von Tensiden in bikontinuierlichen Mikroemulsionen erreicht werden.

Die Lösung besteht in einer Mischung, enthaltend ein Tensid und ein Cotensid, wobei das Cotensid ein amphiphiles Polymer, aufweisend eine oder mehrere hydrophobe Untereinheiten (A) und eine oder mehrere hydrophile Untereinheiten (B) ist, die dadurch gekennzeichnet ist, dass eine oder mehrere hydrophobe Untereinheiten (A) aus einem Polyisobuten- Block erhalten durch kationische Polymerisation mit einer Lewis-Säure gebildet sind, dessen Polyisobuten-Makromoleküle zu mindestens 50 Mol-% terminal angeordnete Doppelbindungen aufweisen.

Es wurde überraschend gefunden, dass sich amphiphile Polymere mit der oben definierten Struktur besonders gut als Cotenside eignen, indem sie die Effizienz von Tensiden steigern und dabei aus großtechnisch und somit preiswert zugänglichen Stoffen auf großtechnischen Umsetzungswegen erhältlich sind. Die erfindungsgemäßen amphiphilen Polymere sind in der Regel technische Gemische aus Substanzen mit einer mehr oder weniger breiten Molekulargewichtsverteilung.

Unter die Begriffe "ein Tensid" beziehungsweise "ein Cotensid" fallen im Rahmen der vorliegenden Erfindung auch jeweils Gemische von Tensiden beziehungsweise von Cotensiden.

Bevorzugt ist jede hydrophobe Untereinheit aus einem Polyisobuten-Block gebildet.

Polyisobutene, die der vorstehenden Definition entsprechen, d.h. die zu mindestens 50 Mol% aus Makromolekülen mit terminal angeordneten Doppelbindungen gebildet sind, werden als sogenannte reaktive Polyisobutene bezeichnet. Dabei werden unter dem Begriff terminal angeordnete Doppelbindungen sowohl β-olefinische (Vinyl-) Doppelbindungen -[-CH=C(CH₃)₂] als auch α-olefinische (Vinyliden-)Doppelbildungen -[-C(CH₃)=CH₂] verstanden. Bevorzugte reaktive Polyisobutene sind solche, bei denen wenigstens 60 Mol%, bevorzugt wenigstens 80 Mol% der Polyisobuten-Makromoleküle, bezogen auf die Gesamtzahl der Polyisobuten-Makromoleküle, terminal angeordnete Doppelbindungen aufweisen.

Geeignete reaktive Polyisobutene können beispielsweise durch kationische Polymerisation von Isobuten erhalten werden.

Zur Synthese geeigneter Polyisobutene setzt man bevorzugt reines Isobuten ein. Es können aber zusätzlich auch kationisch polymerisierbare Comonomere verwendet werden. Die Menge an Comonomeren sollte jedoch im Regelfall weniger als 20 Gew.-%, vorzugsweise weniger als 10 Gew.-% und insbesondere weniger als 5 Gew.-% betragen.

Als kationisch polymerisierbare Comonomere kommen vor allem Vinylaromaten, wie Styrol und α-Methylstyrol, C₁-C₄-Alkylstyrole, sowie 2-,3- und 4-Methylstyrol sowie 4-tert.-Butylstyrol, C₃- bis C₆-Alkene wie n-Buten, Isoolefine mit 5 bis 10 C-Atomen wie 2-Methylbuten-1, 2-Methylpenten-1, 2-Methylhexen-1, 2-Ethylpenten-1, 2-Ethylhexen-1 und 2-Propylhepten-1 in Betracht.

Als Isobuten-haltige Einsatzstoffe für das erfindungsgemäße Verfahren eignen sich sowohl Isobuten selber, als auch Isobuten-haltige C₄-Kohlenwasserstoffströme, beispielsweise C₄-Raffinate, C₄-Schnitte aus der Isobutan-Dehydrierung, C₄-Schnitte aus Steamcrackern oder sogenannten FCC-Crackern (FCC: Fluid Catalysed Cracking), sofern sie weitgehend von darin enthaltenem 1,3-Butadien befreit sind. Typischerweise liegt die Konzentration von Isobuten in C₄-Kohlenwasserstoffströmen im Bereich von 40 bis 60 Gew.-%.

Geeignete C₄-Kohlenwasserstoffströme sollten in der Regel weniger als 500 ppm, vorzugsweise weniger als 200 ppm 1,3-Butadien enthalten. Die Anwesenheit von Buten-1, cis- und trans-Buten-2 ist für die Polymerisation weitgehend unkritisch und führt nicht zu Selektivitätsverlusten.

Bei Einsatz von C₄-Kohlenwasserstoffströmen als Einsatzmaterial übernehmen die von Isobuten verschiedenen Kohlenwasserstoffe die Rolle eines inerten Lösungsmittels oder werden als Comonomer einpolymerisiert.

Als Lösungsmittel kommen alle organischen Verbindungen in Frage, die im gewählten Temperaturbereich der Herstellung der Polyisobutene flüssig sind und weder Protonen abspalten, noch freie Elektronenpaare aufweisen.

Zu nennen sind insbesondere cyclische und acyclische Alkane wie Ethan, iso- und n-Propan, n-Butan und seine Isomeren, Cyclopentan sowie n-Pentan und seine Isomeren, Cyclohexan sowie n-Hexan und seine Isomeren, n-Heptan und seine Isomeren sowie höhere Homologe, cyclische und acyclische Alkene wie Ethen, iso- und n-Propen, n-Buten, Cyclopenten sowie n-Penten, Cyclohexen sowie n-Hexen, n-Hepten, aromatische Kohlenwasserstoffe wie Benzol, Toluol oder isomeren Xylole. Die Kohlenwasserstoffe können auch halogeniert sein. Beispiele halogenierter Kohlenwasserstoffe umfassen Methylchlorid, Methylbromid, Methylenchlorid, Methylenbromid, Ethylchlorid, Ethylbromid, 1,2-Dichlorethan, 1,1,1-Trichlorethan, Chloroform oder Chlorbenzol. Es können auch Gemisch der Lösungsmittel eingewetzt werden, vorausgesetzt, es treten keine unerwünschten Eigenschaften auf.

Verfahrenstechnisch besonders empfehlenswert ist es, Lösungsmittel einzusetzen, die im gewünschten Temperaturbereich sieden. Die Polymerisation erfolgt üblicherweise bei -80°C bis 0°C, bevorzugt -50°C bis -5°C und besonders bevorzugt bei -30°C bis -15°C.

Als Katalysator können reines BF₃, seine Komplexe mit Elektronendonatoren oder Mischungen daraus eingesetzt werden. Elektronendonatoren (Lewis-Basen) sind Verbindungen, die ein freies Elektronenpaar, zum Beispiel an einem O, N, P oder S-Atom, aufweisen und mit Lewis-Säuren Komplexe bilden können. Diese Komplexbildung ist in vielen Fällen erwünscht, da so die Aktivität der Lewis-Säure herabgesetzt wird und Nebenreaktionen zurückgedrängt werden. Beispiele für geeignete Elektronendonatoren sind Ether wie DiIsopropylether oder Tetrahydrofuran, Amine wie Triethylamin, Amide wie DiMethylacetamid, Alkohole wie Methanol, Ethanol, i-Propanol oder t-Butanol. Die Alkohole wirken außerdem als Protonenquelle und starten so die Polymerisation. Auch über Protonen aus ubiquitären Wasserspuren kann ein kationischer Polymerisationsmechanismus aktiv werden.

Bei der kationischen Polymerisation unter BF₃-Katalyse werden weitgehend lineare Polyisobutene erhalten, die an einem Kettenende einen besonders hohen Gehalt an α-OlefinGruppen aufweisen. Bei geeigneter Reaktionsführung beträgt der α-Olefin-Gehalt nicht weniger als 80 %.

Reaktive Polyisobutene, die an beiden Kettenenden reaktive α-Olefin-Gruppen aufweisen oder die verzweigt sind, können besonders elegant mittels lebender kationischer Polymerisation erhalten werden. Selbstverständlich können aber auch lineare Polyisobutene, die nur an einem Kettenende eine α-Olefin-Gruppe aufweisen, mit dieser Methode synthetisiert werden.

Bei der lebenden kationischen Polymerisation wird Isobuten mit einer geeigneten Kombination aus einem Initiatormolekül IXₙ mit einer Lewis-Säure S polymerisiert. Einzelheiten dieser Methode zur Polymerisation sind beispielsweise in Kennedy u. Ivan, "Carbocationic Macromolecular Engineering", Hanser Publishers 1992 offenbart.

Geeignete Initiatormoleküle IXₙ weisen eine oder mehrere Abgangsgruppen X auf. Bei der Abgangsgruppe X handelt es sich um eine Lewis Base, die auch noch weiter substituiert sein kann. Beispiele für geeignete Abgangsgruppen umfassen die Halogene Fluor, Chlor, Brom und Jod, geradkettige und verzweigte Alkoxygruppen, wie C₂H₅O-, n-C₃H₇O-, i-C₃H₇O-, n-C₄H₉O-, i-C₄H₉O-, sec.-C₄H₉O- oder t-C₄H₉O-, sowie geradkettige und verzweigte Carboxygruppen wie CH₃ CO-O-, C₂H₅ CO-O-, n-C₃H₇ CO-O-, 1-C₃H₇ CO-O-, n-C₄H₉ CO-0-, i-C₄H₉ CO-O-, sec.-C₄H₉ CO-O-, t-C₄H₉ CO-O-. Mit der oder den Abgangsgruppen verbunden ist der Molekülteil I, der unter Reaktionsbedingungen ausreichend stabile Carbokationen I⁺ ausbilden kann. Zur Auslösung der Polymerisation wir die Abgangsgruppe mittels einer geeigneten Lewis-Säure S abstrahiert: I-X + S → I⁺ + XS- (gezeigt hier nur für den Fall n = 1). Das entstehende Carbokation I⁺ startet die kationische Polymerisation und wird in das entstehende Polymer eingebaut. Geeignete Lewis-Säuren S sind beispielsweise AIY₃, TiY₄, BY₃, SnY₄, ZnY₂ wobei Y für Fluor, Chlor, Brom oder Jod steht. Die Polymerisationsreaktion kann durch die Vernichtung der Lewis-Säure abgebrochen werden, beispielsweise durch deren Reaktion mit Alkohol. Dabei bildet sich Polyisobuten welches über terminale -C(CH₃)₂-Z Gruppen verfügt, die anschließend in α- und β-Olefin-Endgruppen übergeführt werden können.

Als Initiatormolekül bevorzugt sind Strukturen, die tertiäre Carbokationen ausbilden können. Besonders bevorzugt sind Reste, die sich von den niederen Oligomeren des Isobutens H-[CH₂-C(CH₃)₂]ₙ-X ableiten, wobei n bevorzugt für 2 bis 5 steht. Mit derartigen Initiatormolekülen gebildete lineare reaktive Polyisobutene weisen nur an einem Ende eine reaktive Gruppe auf.

Lineare Polyisobutene, die an beiden Enden reaktive Gruppen aufweisen, können erhalten werden, indem man Initiatormoleküle IXQ einsetzt, die zwei Abgangsgruppen X bzw. Q aufweisen, wobei X und Q gleich oder verschieden sein können. In der Technik bewährt haben sich Verbindungen, die -C(CH₃)₂-X Gruppen umfassen. Beispiele umfassen geradkettige oder verzweigte Alkylenreste CₙH₂ₙ (wobei n vorzugsweise Werte von 4 bis 30 einnehmen kann), die auch von einer Doppelbindung oder einem Aromaten unterbrochen sein können, wie
X-(CH₃)₂C-CH₂-C(CH₃)₂-Q, X-(CH₃)₂C-CH₂-C(CH₃)₂CH₂-C(CH₃)₂-Q,
X-(CH₃)₂C-CH₂-C(CH₃)₂CH₂-C(CH₃)₂CH₂-C(CH₃)₂-Q oder
X-(CH₃)₂C-CH₂-C(CH₃)₂CH₂-C(CH₃)₂CH₂-C(CH₃)₂-CH₂-C(CH₃)₂-Q,
X-(CH₃)₂C-CH=CH-C(CH₃)₂-Q oder para und/oder meta
X-(CH₃)₂C-C₆H₄-C(CH₃)₂-Q.

Verzweigte Polyisobutene können erhalten werden, indem man Initiatormoleküle IXₙ einsetzt, die 3 oder mehrere Abgangsgruppen aufweisen, wobei die Abgangsgruppen gleich oder verschieden sein können. Beispiele geeigneter Initiatormoleküle umfassen X-(CH₃)₂C-C₆H₃-[C(CH₃)₂-Q]-C(CH₃)₂-P als 1,2,4 und/oder 1,3,5-Isomer, wobei die Abgangsgruppen bevorzugt gleich sind, aber auch unterschiedlich sein können. Weitere Beispiele für mono-, di-, tri- oder polyfunktionelle Initiatormoleküle sind in dem eingangs zitierten Werk von Kennedy u. Ivan und der dort zitierten Literatur zu finden.

Geeignete Polyisobutene sind beispielsweise die Glissopal^{®}-Marken der BASF AG, beispielsweise Glissopal 550, 1000, 1300 oder 2300, sowie die Oppanol^{®}-Marken der BASF AG, wie Oppanol B 10 oder B 12.

Für die erfindungsgemäße Mischung sind Cotenside besonders geeignet, die einen Polyisobuten-Block mit einem zahlenmittleren Molekulargewicht Mₙ im Bereich von 200 bis 20000 Dalton, bevorzugt im Bereich von 200 bis 5000 Dalton, aufweisen.

Je nach Polymerisationsverfahren liegt der Polydispersitätsindex (PDI), d.h. das Verhältnis aus gewichtsmittlerem und zahlenmittlerem Molekulargewicht, der vorzugsweise einsetzbaren Polyisobutene im Bereich von 1,05 bis 10, bevorzugt im Bereich von 1,05 bis 5, besonders bevorzugt im Bereich von 1,05 bis 2,0.

Die Methode zur Bestimmung der Polydispersität (PDI) sowie zum zahlenmittleren und gewichtsmittleren Molekulargewicht ist beispielsweise im Analytiker-Taschenbuch, Band 4, Seiten 433 bis 442, Berlin 1984, beschrieben.

Die Erfindung ist grundsätzlich nicht eingeschränkt bezüglich der zur Bildung des Cotensids einsetzbaren einer oder mehreren hydrophilen Untereinheiten.

Besonders vorteilhaft sind Untereinheiten, die in Wasser besonders gut und in Öl besonders schlecht löslich sind.

Bevorzugt sind eine oder mehrere hydrophile Untereinheiten (B) aus sich wiederholenden Ethylenoxid- oder Ethylenoxid-/Propylenoxid-Einheiten gebildet, bevorzugt mit einem Anteil von 0 bis 50 % Propylenoxid-, besonders bevorzugt mit einem Anteil von 5 bis 20 % Propylenoxideinheiten. Hierbei kann es sich um ein statistisches Copolymer, ein Gradienten-Copolymer, ein alternierendes oder ein Blockcopolymer aus Ethylenoxid und Propylenoxid handeln.

Weiter bevorzugt ist eine oder mehrere hydrophile Untereinheit (B) aus Monomereinheiten, ausgewählt aus der nachfolgenden Gruppe: (Meth)acrylsäure, auch teilweise oder vollständig neutralisiert, (Meth)acrylaten, Vinylacetat, Vinylalkohol, Vinylpyrrolidon, Polyallylalkohol, sowie hydrophile Derivate der vorstehend aufgeführten Monomereinheiten oder aus Mischungen hiervon gebildet.

Die das amphiphile Polymer bildenden hydrophoben und hydrophilen Untereinheiten werden bevorzugt verbunden, indem man den Polyisobuten-Block, der die Basis für die hydrophobe Untereinheit(en) bildet, unter Einführung von polaren Gruppen funktionalisiert und den funktionalisierten Polyisobuten-Block anschließend gegebenenfalls weiter modifiziert.

Der Funktionalisierungsgrad der modifizierten Polyisobutyen-Derivate mit terminalen, polaren Gruppen beträgt mindestens 65%, bevorzugt mindestens 75% und ganz besonders bevorzugt mindestens 85%. Bei den nur an einem Kettenende polare Gruppen aufweisenden Polymeren bezieht sich diese Angabe nur auf dieses eine Kettenende. Bei den an beiden Kettenenden polare Gruppen aufweisenden Polymeren sowie den verzweigten Produkten bezieht sich diese Angabe auf die Gesamtzahl aller Kettenenden. Bei den nicht funktionalisierten Kettenenden handelt es sich sowohl um solche die überhaupt keine reaktive Gruppe aufweisen wie um solche, die zwar eine reaktive Gruppe aufweisen, diese aber im Zuge der Funktionalisierungsreaktion nicht umgesetzt wurden.

Der Begriff "polare Gruppe" ist dem Fachmann bekannt. Bei den polaren Gruppen kann es sich sowohl um protische als auch um aprotische polare Gruppen handeln. Die modifizierten Polyisobutene weisen somit einen hydrophoben Molekülteil aus einem Polyisobutenrest auf sowie einen Molekülteil, der zumindest einen gewissen hydrophilen Charakter aufweist, aus terminalen, polaren Gruppen. Bevorzugt handelt es sich um stark hydrophile Gruppen. Die Begriffe "hydrophil" sowie "hydrophob" sind dem Fachmann bekannt.

Polare Gruppen umfassen beispielsweise Sulfonsäurereste, Carbonsäureanhydride, Carboxyl-Gruppen, Carbonsäureamide, Carbonsäureester, Phosphonsäure-Gruppen, Phosphonester und-amide, Hydroxy-Gruppen, Arylhydroxy-Gruppen, Arylphosphorsäureester, A-rylschwefelsäureester, Polyoxyalkylen-Gruppen, Polyoxyalkylenester der genannten Säure-Gruppen, Amino-Gruppen, Polyethylenimino-Gruppen, Amide von Polyethyleniminen der genannten Säuren oder Epoxyde, die auch noch geeignet substituiert sein können.

Geeignete Reaktionen zur Einführung von polaren Gruppen (Funktionalisierung) sind dem Fachmann grundsätzlich bekannt.

Grundsätzlich kann die Funktionalisierung der erfindungsgemäß eingesetzten Polyisobutene ein- oder mehrstufig durchgeführt werden.

In einer bevorzugten Ausführungsform erfolgt die Funktionalisierung des erfindungsgemäß eingesetzten Polyisobutens ein- oder mehrstufig und ist ausgewählt aus:
i) Umsetzung mit aromatischen Hydroxyverbindungen in Gegenwart eines Alkylierungskatalysators unter Erhalt von mit Polyisobutenen alkylierten aromatischen Hydroxyverbindungen,
ii) Umsetzung des Polyisobuten-Blocks mit einer Peroxi-Verbindung unter Erhalt eines epoxidierten Polyisobutens,
iii) Umsetzung des Polyisobuten-Blocks mit einem Alken, das eine mit elektronenziehenden Gruppen substituierte Doppelbindung aufweist (Enophil), in einer En-Reaktion,
iv) Umsetzung des Polyisobuten-Blocks mit Kohlenmonoxid und Wasserstoff in Gegenwart eines Hydroformylierungskatalysators unter Erhalt eines hydroformylierten Polyisobutens,
v) Umsetzung des Polyisobuten-Blocks mit einem Phosphorhalogenid oder einem Phosphoroxychlorid unter Erhalt eines mit Phosphongruppen funktionalisiertem Polyisobutens,
vi) Umsetzung des Polyisobuten-Blocks mit einem Boran und anschließender oxidativer Spaltung unter Erhalt eines hydroxylierten Polyisobutens,
vii) Umsetzung des Polyisobuten-Blocks mit einer SO₃-Quelle, bevorzugt Acetylsulfat oder Oleum unter Erhalt eines Polyisobutens mit terminalen Sulfonsäuregruppen,
viii) Umsetzung des Polyisobuten-Blocks mit Stickoxiden und anschließende Hydrierung unter Erhalt eines Polyisobutens mit terminalen Aminogruppen.

### Zu i): Alkylierung von aromatischen Hydroxyverbindungen

Zur Derivatisierung kann das reaktive Polyisobuten mit einer aromatischen Hydroxyverbindung in Gegenwart eines Alkylierungskatalysators umgesetzt werden. Geeignete Katalysatoren und Reaktionsbedingungen dieser sogenannten Friedel-Crafts-Alkylierung sind beispielsweise in J. March, Advanced Organic Chemistry, 4. Auflage, Verlag John Wiley & Sons, S. 534-539 beschrieben, worauf hier Bezug genommen wird.

Die zur Alkylierung eingesetzte aromatische Hydroxyverbindung ist vorzugsweise ausgewählt unter phenolischen Verbindungen mit 1, 2 oder 3 OH-Gruppen, die gegebenenfalls wenigstens einen weiteren Substituenten aufweisen können. Bevorzugte weitere Substituenten sind C₁-C₈-Alkylgruppen und insbesondere Methyl und Ethyl. Bevorzugt sind insbesondere Verbindungen der allgemeinen Formel, 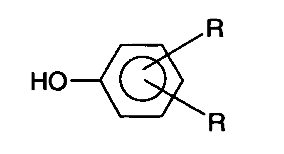 worin R¹ und R² unabhängig voneinander für Wasserstoff, OH oder CH₃ stehen. Besonders bevorzugt sind Phenol, die Kresol-Isomere, Katechol, Resorcinol, Pyrogallol, Fluoroglucinol und die Xylenol-Isomere. Insbesondere werden Phenol, o-Kresol und p-Kresol eingesetzt. Gewünschtenfalls können auch Gemische der zuvor genannten Verbindungen zur Alkylierung eingesetzt werden.

Der Katalysator ist vorzugsweise ausgewählt unter Lewis-sauren Alkylierungskatalysatoren, worunter im Rahmen der vorliegenden Anmeldung sowohl einzelne Akzeptoratome als auch Akzeptor-Ligand-Komplexe, Moleküle, etc. verstanden werden, sofern diese insgesamt (nach außen) Lewis-saure (Elektronenakzeptor-)Eigenschaften aufweisen. Dazu zählen beispielsweise AlCl₃, AlBr₃, BF₃, BF₃2 C₆H₅OH, BF₃[O(C₂H₅)₂]₂, TiCl₄, SnCl₄, AlC₂H₅Cl₂, FeCl₃, SbCl₅ und SbF₅. Diese Alkylierungskatalysatoren können gemeinsam mit einem Cokatalysator, beispielsweise einem Ether, eingesetzt werden. Geeignete Ether sind Di-(C₁-C₈-)alkylether, wie Dimethylether, Diethylether, Di-n-propylether, sowie Tetrahydrofuran, Di-(C₅-C₈-)cycloalkylether, wie Dicyclohexylether und Ether mit mindestens einem aromatischen Kohlenwasserstoffrest, wie Anisol. Wird zur Friedel-Crafts-Alkylierung ein Katalysator-Cokatalysator-Komplex eingesetzt, so liegt das Molmengenverhältnis von Katalysator zu Cokatalysator vorzugsweise in einem Bereich von 1:10 bis 10:1. Die Reaktion kann auch mit Protonensäuren wie Schwefelsäure, Phosphorsäure, Trifluormethansulfonsäure katalysiert werden. Organische Protonensäuren können auch in polymer gebundener Form vorliegen, beispielsweise als Ionenaustauscherharz.

Die Alkylierung kann lösungsmittelfrei oder in einem Lösungsmittel durchgeführt werden. Geeignete Lösungsmittel sind beispielsweise n-Alkane und deren Gemische und Alkylaromaten, wie Toluol, Ethylbenzol und Xylol sowie halogenierte Abkömmlinge davon.

Die Alkylierung wird bevorzugt bei Temperaturen zwischen -10°C und +100°C durchgeführt. Die Reaktion wird üblicherweise bei Atmosphärendruck durchgeführt, kann aber auch bei höheren oder geringeren Drücken durchgeführt werden.

Durch geeignete Wahl der Molmengenverhältnisse von aromatischer Hydroxyverbindung zu Polyisobuten und des Katalysators kann der erzielte Anteil an alkylierten Produkten und deren Alkylierungsgrad eingestellt werden. So werden z. B im Wesentlichen monoalkylierte Polyisobutenylphenole im Allgemeinen mit einem Überschuss an Phenol oder in Gegenwart eines Lewis-sauren Alkylierungskatalysators erhalten, wenn zusätzlich ein Ether als Cokatalysator eingesetzt wird.

Die Reaktion von Polyisobutenen mit Phenolen in Anwesenheit geeigneter Alkylierungskatalysatoren ist z. B. in US 5,300,701 und WO 02/26840 offenbart.

Zur weiteren Funktionalisierung kann man ein in Schritt i) erhaltenes Polyisobutenylphenol einer Umsetzung im Sinne einer Mannichreaktion mit wenigstens einem Aldehyd, beispielsweise Formaldehyd, und wenigstens einem Amin, das wenigstens eine primäre oder sekundäre Aminfunktion aufweist, unterziehen, wobei man eine mit Polyisobuten alkylierte und zusätzlich wenigstens teilweise aminoalkylierte Verbindung erhält. Es können auch Reaktions- und/oder Kondensationsprodukte von Aldehyd und/oder Amin eingesetzt werden. Die Herstellung solcher Verbindungen sind in WO 01/25 293 und WO 01/25 294 beschrieben, auf die hiermit im vollen Umfang Bezug genommen wird.

Des Weiteren kann ein in Schritt i) erhaltenes Polyisobutenylphenol mit Alkylenoxiden, bevorzugt Ethylenoxid, alkoxyliert werden. Neben Ethylenoxid können nachfolgende reine Alkylenoxide oder auch Mischungen verwendet werden: Propenoxid, 1-Butenoxid 2,3-Butenoxid, 2-Methyl-1,2-propenoxid (Isobutenoxid), 1-Pentenoxid, 2,3-Pentenoxid, 2-Methyl-1,2-butenoxid, 3-Methyl-1,2-butenoxid, 2,3-Hexenoxid, 3,4-Hexenoxid, 2-Methyl-1,2-pentenoxid, 2-Ethyl-1,2-butenoxid, 3-Methyl-1,2-Pentenoxid, Decenoxid, 4-Methyl-1,2-pentenoxid, Styroloxid oder aus einer Mischung aus Oxiden technisch verfügbarer Raffinatströme gebildet sein.

In einer weiteren Ausführungsform werden die erhaltenen Polyisobutenylphenole mit Phosphoroxychlorid zum aromatischen Phosphorhalbester umgesetzt. Dieser wird in einem Folgeschritt mit Polyethyleniminen, Alkylenoxiden oder Polyalkylenoxiden umgesetzt.

### ii) Epoxidierung

Zur Funktionalisierung kann das reaktive Polyisobuten mit wenigstens einer Peroxiverbindung unter Erhalt eines epoxidierten Polyisobutens umgesetzt werden. Geeignete Verfahren zur Epoxidierung sind in J. March, Advanced Organic Chemistry, 4. Auflage, Verlag John Wiley & Sons, S.826-829 beschrieben, worauf hier Bezug genommen wird. Vorzugsweise wird als Peroxiverbindung wenigstens eine Persäure, wie m-Chlorperbenzoesäure, Perameisensäure, Peressigsäure, Trifluorperessigsäure, Perbenzoesäure und 3,5-Dinitroperbenzoesäure eingesetzt. Die Herstellung der Persäuren kann in situ aus den entsprechenden Säuren und H₂O₂ gegebenenfalls in Gegenwart von Mineralsäuren erfolgen. Weitere geeignete Epoxidierungsreagenzien sind beispielsweise alkalisches Wasserstoffperoxid, molekularer Sauerstoff und Alkylperoxide, wie tert.-Butylhydroperoxid. Geeignete Lösungsmittel für die Epoxidierung sind beispielsweise übliche, nicht polare Lösungsmittel. Besonders geeignete Lösungsmittel sind Kohlenwasserstoffe wie Toluol, Xylol, Hexan oder Heptan.

Zur weiteren Funktionalisierung können die epoxidierten Polyisobutene, die in Schritt ii) erhalten werden, mit Ammoniak umgesetzt werden, wobei Polyisobutenaminoalkohole erhalten werden (EP-A 0 476 785).

In einem weiteren Schritt werden die erhaltenen epoxidierten Polyisobutene mit genannten Alkylenoxiden umgesetzt. Bevorzugt ist dabei Ethylenoxid.

### iii) En-Reaktion

Zur Funktionalisierung kann das reaktive Polyisobuten des Weiteren mit wenigstens einem Alken, das eine elektronenarme Doppelbindung aufweist, in einer En-Reaktion umgesetzt werden (siehe z. B. DE-A 4 319 672 oder H. Mach und P. Rath in "Lubrication Science II (1999), S. 175-185, worauf voll inhaltlich Bezug genommen wird). Bei der En-Reaktion wird ein als En bezeichnetes Alken mit einem Allyl-ständigen Wasserstoffatom mit einem elektronenarmen Alken, dem sogenannten Enophil, in einer pericyclischen Reaktion, umfassend eine Kohlenstoff-Kohlenstoff-Bindungsknüpfung, eine Doppelbindungsverschiebung und einen Wasserstofftransfer umgesetzt. Vorliegend reagiert das reaktive Polyisobuten als En. Geeignete Enophile sind Verbindungen, wie sie auch als Dienophile in der Diels-Alder-Reaktion eingesetzt werden. Als Enophile eignen sich Fumarsäuredichlorid, Fumarsäure, Maleinsäuredichlorid, Maleinsäureanhydrid und Maleinsäure, bevorzugt Maleinsäureanhydrid und Maleinsäure. Gans besonders bevorzugt wird als Enophil Maleinsäureanhydrid eingesetzt. Dabei resultieren mit Bernsteinsäureanhydridgruppen (Succinanhydridgruppen) funktionalisierte Polyisobutene (Polyisobutenylbernsteinsäureanhydrid, PIBSA), wie in EP-A 0 156 310 offenbart.

Die En-Reaktion kann gegebenenfalls in Gegenwart einer Lewis-Säure als Katalysator durchgeführt werden. Geeignet sind beispielsweise Aluminiumchlorid und Ethylaluminiumchlorid.

Bei der Umsetzung wird eine neue α-Olefingruppe am Kettenende erzeugt. Zur weiteren Funktionalisierung kann man beispielsweise ein mit Bernsteinsäureanhydridgruppen derivatisiertes Polyisobuten einer Folgereaktion unterziehen, die ausgewählt ist unter:
a) Umsetzung mit wenigstens einem Amin unter Erhalt eines wenigstens teilweise mit Succinimidgruppen und/oder Succinamidgruppen funktionalisierten Polyisobutens,
b) Umsetzung mit wenigstens einem Alkohol unter Erhalt eines mit Succinestergruppen funktionalisierten Polyisobutens,
c) Umsetzung mit wenigstens einem Alkylenoxid unter Erhalt eines mit zwei Succinestergruppen (pro Bernsteinsäureanhydrid-Gruppe) funktionalisierten Polyisobutens,
d) Umsetzung mit Maleinsäureanhydrid zu einem Produkt mit zwei Bemsteinsäureanhydrid-Gruppen am Kettenende (sogenannte PIBBSA).
e) Hydrolyse unter Erhalt eines mit Bernsteinsäuregruppen funktionalisierten Polyisobutens, wobei die Bernsteinsäuregruppen wie unter c) mit Alkylenoxiden umgesetzt werden,
f) sofern nach der Umsetzung der Bernsteinsäureanhydrid-Gruppe noch freie CarboxylGruppen vorhanden sind, können diese auch in Salze umgewandelt werden. Als Kationen in Salzen kommen vor allem Alkalimetallkationen, Ammonium-ionen sowie Alkylammoiniumionen in Frage.

### Zu a) und b)

Die Bernsteinsäureanhydridgruppen können zur weiteren Derivatisierung beispielsweise mit polaren Reaktionspartnern wie Alkoholen oder Aminen umgesetzt werden. Bevorzugt handelt es sich bei geeigneten polaren Reaktionspartnern um primäre Alkohole ROH oder primäre Amine RNH₂ bzw. sekundäre Amine RR'NH, wobei R für einen linearen oder verzweigten gesättigten Kohlenwasserstoffrest steht, der mindestens einen Substituenten ausgewählt aus der Gruppe OH, NH₂ oder NH₃⁺ und gegebenenfalls ein oder mehrere CH(O)-Gruppen trägt und gegebenenfalls nicht benachbarte -O- und/oder -NH- und/oder tertiäre -N- Gruppen aufweist, und R' unabhängig von R dieselbe Bedeutung hat. Hierbei können beide Carbonsäuregruppen des Bernsteinsäureanhydrids zur Umsetzung kommen oder auch nur eine, während die andere Carbonsäuregruppe als freie Säuregruppe oder als Salz vorliegt. Die obigen Substituenten können auch noch weiter, beispielsweise durch Alkoxylierung modifiziert werden.

Weitere Synthesevarianten für die Derivatisierung von Bernsteinsäureanhydridgruppen sind in den Anmeldungen mit den Aktenzeichen DE 101 251 58.0 und DE 101 476 50.7 genannt.

Es ist dem Fachmann auch bekannt, eine Bernsteinsäureanhydridgruppe unter geeigneten Bedingungen in eine Bernsteinsäureimid-Gruppe überzuführen.

In einer weiteren Ausführungsform kann reaktives Polyisobuten mit Maleinsäureanhydrid radikalisch copolymerisiert werden (vgl. WO 95/07944, WO 01/55059, WO 90/03359). Die so erhaltenen streng alternierenden Copolymere können wie oben beschrieben weiter umgesetzt werden. Bevorzugt sind die Umsetzungen mit Alkylenoxiden, Polyalkylenoxiden oder Polyethyleniminen.

### iv) Hydroformylierung

Zur Funktionalisierung kann man das reaktive Polyisobuten einer Umsetzung mit Kohlenmonoxid und Wasserstoff in Gegenwart eines Hydroformylierungskatalysators unterziehen, wobei ein hydroformyliertes Polyisobuten erhalten wird.

Geeignete Katalysatoren für die Hydroformylierung sind bekannt und umfassen vorzugsweise eine Verbindung oder einen Komplex eines Elements der VIII. Nebengruppe des Periodensystems, wie Co, Rh, Ir, Ru, Pd oder Pt. Zur Aktivitäts- und/oder Selektivitätsbeeinflussung werden vorzugsweise mit N- oder P-haltigen Liganden modifizierte Hydroformylierungskatalysatoren eingesetzt. Geeignete Salze dieser Metalle sind beispielsweise die Hydride, Halogenide, Nitrate, Sulfate, Oxide, Sulfide oder die Salze mit Alkyl- oder Arylcarbonsäuren oder Alkyl- oder Arylsulfonsäuren. Geeignete Komplexverbindungen weisen Liganden auf, die beispielsweise ausgewählt sind unter Halogeniden, Aminen, Carboxylaten, Acetylacetonat, Aryl- oder Alkylsulfonaten, Hydrid, CO, Olefinen, Dienen, Cycloolefinen, Nitrilen, N-haltigen Heterocyclen, Aromaten und Heteroaromaten, Ethern, PF₃, Phospholen, Phosphabenzolen sowie ein-, zwei- und mehrzähnigen Phosphin-, Phosphinit-, Phosphonit-, Phosphoramidit und Phosphitliganden.

Im Allgemeinen werden unter Hydroformylierungsbedingungen aus den jeweils eingesetzten Katalysatoren oder Katalysatorvorstufen katalytisch aktive Spezies der allgemeinen Formel HₓM_{y}(CO)_{z}L_{q} gebildet, worin M für ein Metall der VIII. Nebengruppe, L für einen Liganden und q, x, y, z für ganze Zahlen, abhängig von der Wertigkeit und Art des Metalls sowie der Bindigkeit des Liganden L, stehen.

Nach einer bevorzugten Ausführungsform werden die Hydroformylierungskatalysatoren in situ in dem für die Hydroformylierungsreaktion eingesetzten Reaktor hergestellt.

Eine andere bevorzugte Form ist die Verwendung eines Carbonylgenerators, bei dem vorgefertigtes Carbonyl z. B. an Aktivkohle adsorbiert wird und nur das desorbierte Carbonyl der Hydroformylierung zugeführt wird, nicht aber die Salzlösungen, aus denen das Carbonyl erzeugt wird.

Als Katalysatoren geeignete Rhodiumverbindungen oder -komplexe sind z. B. Rhodium(II)- und Rhodium(III)-salze, wie Rhodium(III)-chlorid, Rhodium(III)-nitrat, Rhodium(III)-sulfat, Kalium-Rhodiumsulfat, Rhodium(II)- bzw. Rhodium(III)-carboxylat, Rhodium(II)- und Rhodium(III)-acetat, Rhodium(III)-oxid, Salze der Rhodium(III)- säure, Trisammoniumhexachlororhodat(III) etc. Weiterhin eignen sich Rhodiumkomplexe, wie Rhodiumbiscarbonylacetylacetonat, Acetylacetonatobisethylenrhodium(I) etc.

Ebenfalls geeignet sind Rutheniumsalze oder -verbindungen. Geeignete Rutheniumsalze sind beispielsweise Ruthenium(III)chlorid, Ruthenium(IV)-, Ruthenium(VI)- oder Ruthenium(VIII)oxid, Alkalisalze der Rutheniumsauerstoffsäuren wie K₂RuO₄ oder KRuO₄ oder Komplexverbindungen, wie z. B. RuHCl(CO)(PPh₃)₃. Auch können die Metallcarbonyle des Rutheniums wie Trisrutheniumdodecacarbonyl oder Hexarutheniumoctadecacarbonyl, oder Mischformen, in denen CO teilweise durch Liganden der Formel PR₃ ersetzt sind, wie Ru(CO)₃(PPh₃)₂, verwendet werden.

Geeignete Cobaltverbindungen sind beispielsweise Cobalt(II)chlorid, Cobalt(II)sulfat, Cobalt(II)carbonat, Cobalt(II)nitrat, deren Amin- oder Hydratkomplexe, Cobaltcarboxylate, wie Cobaltformiat, Cobaltacetat, Cobaltethylhexanoat, Cobaltnaphthanoat, sowie der Cobalt-Caprolactamat-Komplex. Auch hier können die Carbonylkomplexe des Cobalts wie Dicobaltoctacarbonyl, Tetracobaltdodecacarbonyl und Hexacobalthexadecacarbonyl eingesetzt werden.

Die genannten und weiteren geeignete Verbindungen sind im Prinzip bekannt und in der Literatur hinreichend beschrieben.

Geeignete Aktivierungsmittel, die zur Hydroformylierung eingesetzt werden können, sind z. B. Brönsted-Säuren, Lewis-Säuren, wie BF₃, AlCl₃, ZnCl₂, und Lewis-Basen.

Die Zusammensetzung des eingesetzten Synthesegases aus Kohlenmonoxid und Wasserstoff kann in weiten Bereichen variieren. Das molare Verhältnis von Kohlenmonoxid und Wasserstoff beträgt in der Regel etwa 5:95 bis 95:5, bevorzugt etwa 40:60 bis 60:40. Die Temperatur bei der Hydroformylierung liegt im Allgemeinen in einem Bereich von etwa 20 bis 200°C, bevorzugt etwa 50 bis 190°C. Die Reaktion wird in der Regel bei dem Partialdruck des Reaktionsgases bei der gewählten Reaktionstemperatur durchgeführt. Im Allgemeinen liegt der Druck in einem Bereich von etwa 1 bis 700 bar, bevorzugt 1 bis 300 bar.

Die Carbonylzahl der erhaltenen hydroformylierten Polyisobutene hängt vom zahlenmittleren Molekulargewicht Mₙ ab. Vorzugsweise weisen Produkte mit einem zahlenmittleren Molekulargewicht Mₙ von 10000 Dalton Carbonylzahlen von 2 bis 5,6 mg KOH/g, insbesondere 3,6 bis 5,6 mg KOH/g auf. Produkte mit einem zahlenmittleren Molekulargewicht Mₙ von 40000 Dalton weisen Carbonylzahlen von 0,5 bis 1,4 mg KOH/g, insbesondere 0,9 bis 1,4 mg KOH/g auf. Die Carbonylzahlen für Produkte mit anderen Molekulargewichten lassen sich durch Inter- oder Extrapolation bestimmen.

Vorzugsweise wird der überwiegende Teil der in dem eingesetzten mittelmolekularen, reaktiven Polyisobuten enthaltenen Doppelbindungen durch die Hydroformylierung in Aldehyde überführt. Durch Einsatz geeigneter Hydroformylierungskatalysatoren und/oder eines Überschusses an Wasserstoff im eingesetzten Synthesegas kann der überwiegende Teil der im Edukt enthaltenen, ethylenisch ungesättigten Doppelbindungen auch direkt in Alkohole umgewandelt werden (siehe z. B. DE-A 100 03 105). Dies kann auch in einer zweistufigen Funktionalisierung gemäß dem im Folgenden beschriebenen Reaktionsschritt B) erfolgen.

Die durch Hydroformylierung erhaltenen funktionalisierten Polyisobutene eignen sich vorteilhaft als Zwischenprodukte für die Weiterverarbeitung durch Funktionalisierung wenigstens eines Teils der in ihnen enthaltenen Aldehydfunktionen.

### A) Oxocarbonsäuren

Zur weiteren Funktionalisierung kann man die in Schritt iv) erhaltenen hydroformylierten Polyisobutene mit einem Oxidationsmittel unter Erhalt eines wenigstens teilweise mit Carboxygruppen funktionalisierten Polyisobutens umsetzen.

Für die Oxidation von Aldehyden zu Carbonsäuren können allgemein eine große Anzahl verschiedener Oxidationsmittel und -verfahren verwendet werden, die z. B. in J. March, Advanced Organic Chemistry, Verlag John Wiley & Sons, 4. Auflage, S. 701ff. (1992) beschrieben sind. Dazu zählen z. B. die Oxidation mit Permanganat, Chromat, Luftsauerstoff, etc. Die Oxidation mit Luft/Sauerstoff kann sowohl katalytisch in Gegenwart von Metallsalzen als auch in Abwesenheit von Katalysatoren erfolgen. Als Metalle werden bevorzugt solche verwendet, die zu einem Wertigkeitswechsel befähigt sind, wie Cu, Fe, Co, Mn, etc. Die Reaktion gelingt in der Regel auch in Abwesenheit eines Katalysators. Bei der Luftoxidation kann der Umsatz leicht über die Reaktionsdauer gesteuert werden.

Nach einer weiteren Ausführungsform wird als Oxidationsmittel eine wässrige Wasserstoffperoxidlösung in Kombination mit einer Carbonsäure, wie z. B. Essigsäure, verwendet. Die Säurezahl der erhaltenen Polyisobutene mit Carboxylfunktion hängt vom zahlenmittleren Molekulargewicht Mₙ ab. Vorzugsweise weisen Produkte mit einem zahlenmittleren Molekulargewicht Mₙ von 10000 Dalton Säurezahlen von 2 bis 5,6 mg KOH/g, insbesondere 3,6 bis 5,6 mg KOH/g auf. Produkte mit einem zahlenmittleren Molekulargewicht Mₙ von 40000 Dalton weisen Säurezahlen von 0,5 bis 1,4 mg KOH/g, insbesondere 0,9 bis 1,4 mg KOH/g auf. Die Säurezahlen für Produkte mit anderen Molekulargewichten lassen sich durch Inter- oder Extrapolation bestimmen.

Die erhaltenen Polyisobutene mit Carboxyfunktion können in einem weiteren Reaktionsschritt umgesetzt werden. Umsetzungen können sein mit Alkylenoxiden, Veresterungen mit Polyalkylenoxiden oder Amidbildungen mit Polyethyleniminen.

### B) Oxoalkohole

Nach einer weiteren geeigneten Ausführungsform können die in Schritt iv) erhaltenen hydroformylierten Polyisobutene einer Umsetzung mit Wasserstoff in Gegenwart eines Hydrierkatalysators unter Erhalt eines wenigstens teilweise mit Alkoholgruppen funktionalisierten Polyisobutens unterzogen werden.

Geeignete Hydrierungskatalysatoren sind im allgemeinen Übergangsmetalle wie Cr, Mo, W, Fe, Rh, Co, Ni, Pd, Pt, Ru, etc., oder deren Mischungen, die zur Erhöhung der Aktivität und Stabilität auf Trägern, wie Aktivkohle, Aluminiumoxid, Kieselgur, etc., aufgebracht werden können. Zur Erhöhung der katalytischen Aktivität können Fe, Co, und bevorzugt Ni auch in Form der Raney-Katalysatoren als Metallschwamm mit einer sehr großen Oberfläche verwendet werden.

Die Hydrierung der Oxo-Aldehyde aus Stufe iv) erfolgt in Abhängigkeit von der Aktivität des Katalysators vorzugsweise bei erhöhten Temperaturen und erhöhtem Druck. Bevorzugt liegt die Reaktionstemperatur bei etwa 80 bis 150°C und der Druck bei etwa 50 bis 350 bar.

Die Alkoholzahl der erhaltenen Polyisobutene mit Hydroxygruppen hängt vom zahlenmittleren Molekulargewicht Mₙ ab. Vorzugsweise weisen Produkte mit einem zahlenmittleren Molekulargewicht Mₙ von 10000 Dalton Alkoholzahlen von 2 bis 5,6 mg KOH/g, insbesondere 3,6 bis 5,6 mg KOH/g auf. Produkte mit einem zahlenmittleren Molekulargewicht Mₙ von 40000 Dalton weisen Alkoholzahlen von 0,5 bis 1,4 mg KOH/g, insbesondere 0,9 bis 1,4 mg KOH/g auf. Die Alkoholzahlen für Produkte mit anderen Molekulargewichten lassen sich durch Inter- oder Extrapolation bestimmen.

Die mit Alkoholgruppen funktionalisierten Polyisobutene können zusätzlich mit Alkylenoxiden, bevorzugt Ethylenoxid, alkoxyliert werden.

### C) Aminsynthese

Nach einer weiteren geeigneten Ausführungsform werden die in Schritt iv) erhaltenen hydroformylierten Polyisobutene zur weiteren Funktionalisierung einer Umsetzung mit Wasserstoff und Ammoniak oder einem primären oder sekundären Amin in Gegenwart eines Aminierungskatalysators unter Erhalt eines wenigstens teilweise mit Amingruppen funktionalisierten Polyisobutens unterzogen.

Geeignete Aminierungskatalysatoren sind die zuvor in Stufe B) beschriebenen Hydrierungskatalysatoren, bevorzugt Kupfer, Cobalt oder Nickel, die in Form der Raney-Metalle oder auf einem Träger eingesetzt werden können. Weiter eignen sich auch Platinkatalysatoren.

Bei der Aminierung mit Ammoniak werden aminierte Polyisobutene mit primären Aminofunktionen erhalten. Zur Aminierung geeignete primäre und sekundäre Amine sind Verbindungen der allgemeinen Formeln R-NH₂ und RR'NH, worin R und R' unabhängig voneinander beispielsweise für C₁-C₁₀-Alkyl, C₆-C₂₀-Aryl, C₇-C₂₀-Arylalkyl, C₇-C₂₀-Alkylaryl oder Cycloalkyl stehen.

Die Aminzahl der erhaltenen Polyisobutene mit Aminofunktion hängt vom zahlenmittleren Molekulargewicht Mₙ ab. Vorzugsweise weisen Produkte mit einem zahlenmittleren Molekulargewicht Mₙ von 10000 Dalton Aminzahlen von 2 bis 5,6 mg KOH/g, insbesondere 3,6 bis 5,6 mg KOH/g auf. Produkte mit einem zahlenmittleren Molekulargewicht Mₙ von 40000 Dalton weisen Aminzahlen von 0,5 bis 1,4 mg KOH/g, insbesondere 0,9 bis 1,4 mg KOH/g auf. Die Aminzahlen für Produkte mit anderen Molekulargewichten lassen sich durch Inter- oder Extrapolation bestimmen.

Die mit Aminogruppen funktionalisierten Polyisobutene können zusätzlich mit Alkylenoxiden, bevorzugt Ethylenoxid, alkoxyliert werden.

### v) Herstellung von Phosphonsäurederivaten

Zur Funktionalisierung kann das reaktive Polyisobuten einer Umsetzung mit PX₅ (X = Cl, Br, I) unter Erhalt eines mit einer Phosphonsäurehalogenid-Gruppe funktionalisierten Polyisobutens unterzogen werden. Zur weiteren Funktionalisierung und somit zur Ermöglichung einer Pfropfung wird das derivatisierte Polyisobuten einer Folgereaktion unterzogen, die ausgewählt ist unter:
a) Umsetzung mit wenigstens einem Amin oder Polyethylenimin unter Erhalt eines wenigstens teilweise mit Phosphonamidamid-Gruppen funktionalisierten Polyisobutens,
b) Umsetzung mit wenigstens einem Alkohol bzw. Polyalkylenoxid unter Erhalt eines mit Phosphonester-Gruppen funktionalisierten Polyisobutens,
c) Umsetzung mit wenigstens einem Alkylenoxid unter Erhalt eines mit Phosphonester-Gruppen funktionalisierten Polyisobutens,
d) Hydrolyse unter Erhalt eines mit Phosphonsäure-Gruppen funktionalisierten Polyisobutens, wobei die Phosphonsäure-Gruppen wie unter c) mit Alkylenoxiden umgesetzt werden,
f) sofern nach der Umsetzung der Phosphonsäurehalogenid-Gruppe noch freie Säure-Gruppen vorhanden sind, können diese auch in Salze umgewandelt werden. Als Kationen in Salzen kommen vor allem Alkalimetallkationen, Ammonium-ionen sowie Alkylammoniumionen in Frage.

### vi) Hydroborierung

Zur Funktionalisierung kann man das reaktive Polyisobuten einer Umsetzung mit einem (gegebenenfalls in situ erzeugten) Boran unterziehen, wobei ein hydroxyliertes Polyisobuten erhalten wird.

Geeignete Verfahren zur Hydroborierung sind in J. March, Advanced Organic Chemistry, 4. Auflage, Verlag John Wiley & Sons, S. 783-789 beschrieben, worauf hiermit Bezug genommen wird. Geeignete Hydroborierungsreagenzien sind beispielsweise Diboran, das in der Regel in situ durch Umsetzung von Natriumborhydrid mit BF₃-Etherat erzeugt wird, Diisamylboran (Bis-[3-methylbut-2-yl]boran), 1,1,2-Trimethylpropylboran, 9-Borbicyclo[3.3.1]nonan, Diisocampheylboran, die durch Hydroborierung der entsprechenden Alkene mit Diboran erhältlich sind, Chlorboran-Dimethylsulfid, Alkyldichlorborane oder H₃B-N(C₂H₅)₂.

Üblicherweise führt man die Hydroborierung in einem Lösungsmittel durch. Geeignete Lösungsmittel für die Hydroborierung sind beispielsweise acyclische Ether wie Diethylether, Methyl-tert.-butylether, Dimethoxyethan, Diethylenglykoldimethylether, Triethylenglykoldimethylether, cyclische Ether wie Tetrahydrofuran oder Dioxan sowie Kohlenwasserstoffe wie Hexan oder Toluol oder Gemische davon. Die Reaktionstemperatur wird in der Regel von der Reaktivität des Hydroborierungsmittels bestimmt und liegt normalerweise zwischen dem Schmelz- und Siedepunkt des Reaktionsgemisches, vorzugsweise im Bereich von 0°C bis 60°C.

Üblicherweise setzt man das Hydroborierungsmittel im Überschuss bezogen auf das Alken ein. Das Boratom addiert sich vorzugsweise an das weniger substituierte und somit sterisch weniger gehinderte Kohlenstoffatom.

Üblicherweise werden die gebildeten Alkylborane nicht isoliert, sondern durch nachfolgende Umsetzung direkt in die Wertprodukte überführt. Eine sehr bedeutsame Umsetzung der Alkylborane ist die Reaktion mit alkalischem Wasserstoffperoxid unter Erhalt eines Alkohols, der vorzugsweise formal der anti-Markovnikov-Hydroxylierung des Alkens entspricht.

Die mit Alkoholgruppen funktionalisierten Polyisobutene können zusätzlich mit Alkylenoxiden, bevorzugt Ethylenoxid, alkoxyliert werden.

### vii) Umsetzung mit einer SO₃-Quelle

Zur Funktionalisierung kann das reaktive Polyisobuten des Weiteren mit einer SO₃-Quelle umgesetzt werden, wobei ein Polyisobuten mit terminalen Sulfonsäuregruppen gebildet wird.

Die mit Sulfonsäuregruppen funktionalisierten Polyisobutene können durch Umsetzung der reaktiven Polyisobutene mit einer SO₃-Quelle hergestellt werden. Geeignete SO₃-Quellen sind ein Mischung aus Schwefeltrioxid und Luft, Schwefeltrioxid-Hydrate, Schwefeltrioxidaminkomplexe, Schwefeltrioxidetherkomplexe, Schwefeltrioxidphosphatkomplexe, Oleum, Acetylsulfat, eine Mischung von Schwefeltrioxid und Essigsäureanhydrid, Sulfaminsäure, Alkylsulfate oder Chlorsulfonsäuren. Die Reaktion kann entweder in Substanz oder in einem beliebigen inerten wasserfreien Lösungsmittel erfolgen. Geeignete Reaktionstemperaturen liegen im Bereich von -30°C bis +200°C und sind von dem eingesetzten Sulfonierungsreagenz abhängig. Beispielsweise erfolgt eine Sulfonierung mit Acetylsulfat bei niedrigen Temperaturen und erhöhte Temperaturen sollten vermieden werden, da sonst eine Zersetzung des Produktes eintreten kann. Das Sulfonierungsreagenz wird im Allgemeinen in einem Molverhältnis zu Polyisobuten von 1:1 1 bis 2:1 eingesetzt. Bevorzugt wird Acetylsulfat oder eine Mischung von Schwefelsäure und Essigsäureanhydrid, wobei Acetylsulfat in situ gebildet wird, eingesetzt, wobei direkt das mit Sulfonsäuregruppen funktionalisierte Polyisobuten gebildet wird. Andere der genannten Sulfonierungsreagenzien, z. B. die Mischung aus Schwefeltrioxid und Sauerstoff, können zunächst ein intermediäres Sulton bilden, das zur gewünschten Sulfonsäure hydrolysiert werden muss. Ein Verfahren zur Herstellung von mit Sulfonsäuregruppen funktionalisierten Polyisobutenen ist beispielsweise in WO 01/70830 offenbart.

Die mit Sulfonsäure-Gruppen funktionalisierten Polyisobutene werden mit Alkylenoxiden, Polyalkylenoxiden oder Polyethyleniminen umgesetzt.

Sind nach der Funktionalisierung noch freie Säure-Gruppen vorhanden, können diese noch in die Salz-Form überführt werden. Als Kationen in Salzen kommen vor allem Alkalimetallkationen, Ammonium-ionen sowie Alkylammoniumionen in Frage.

### viii) Funktionalisierung mit Aminogruppen

Zur Funktionalisierung kann das reaktive Polyisobuten mit Stickoxiden umgesetzt werden, wobei nach anschließender Hydrierung Polyisobutene mit terminalen Aminogruppen erhalten werden.

Geeignete Stickoxide sind z. B. NO, NO₂, N₂O₃, N₂O₄, Mischungen dieser Stickoxide untereinander und Mischungen dieser Stickoxide mit Sauerstoff. Besonders bevorzugt sind Mischungen von NO oder NO₂ mit Sauerstoff. Des Weiteren können die Stickoxide zusätzlich Inertgase, z. B. Stickstoff, enthalten. Die Umsetzung der Polyisobutene mit den Stickoxiden erfolgt im Allgemeinen bei einer Temperatur von -30 bis +150°C in einem inerten organischen Lösungsmittel. Die erhaltenen Produkte werden anschließend hydriert, bevorzugt durch katalytische Hydrierung mit Wasserstoff in Anwesenheit von Hydrierkatalysatoren. Die Hydrierung wird im Allgemeinen in einem Temperaturbereich von 20 bis 250°C durchgeführt, in Abhängigkeit von dem eingesetzten Reduktionssystem. Der Hydrierdruck beträgt in der katalytischen Hydrierung im Allgemeinen 1 bar bis 300 bar. Ein Verfahren zur Herstellung von mit Aminogruppen terminierten Polymerisaten ist z. B. in WO 97/03946 offenbart.

Die mit Amino-Gruppen funktionalisierten Polyisobutene können zusätzlich mit Alkylenoxiden, bevorzugt Ethylenoxid, alkoxyliert werden.

Werden Säuregruppen (Carbonsäure-, Phosphonsäure-, Phosphorsäure-, Schwefel-säure-Gruppen) mit Polyalkylenoxiden umgesetzt, können Polyalkylenoxide verwendet werden wie zum Beispiel Polyethylenoxid, Polypropylenoxid, gemischte Copolymere aus EO und PO, Monoalkylpolyethylenoxid (Alkyl = Methyl-, Ethyl-, C₁₂-, C₁₈-, etc.), Monoaminoethylenoxid etc.. Bei den Umsetzungen der Säure-Gruppen mit diesen Alkylenoxiden handelt es sich um sogenannte polymeranaloge Reaktionen (Veresterungen). Die Länge der Alkylenoxidkette kann 3 bis 400 Einheiten betragen.

Die in den erfindungsgemäßen Mischungen einzusetzenden Cotenside weisen bevorzugt eine AB-Struktur auf.

Weitere bevorzugte Strukturen des Cotensids sind AₚB_{q} mit p und q unabhängig voneinander von 1 bis 8 oder Kammstrukturen aus A und B.

Die erfindungsgemäße Mischung umfasst neben den vorstehend beschriebenen Co-Tensiden ein Tensid. Dabei kann es sich auch um eine Mischung von Tensiden handeln. Grundsätzlich kann jedes Tensid, aus jeder der bekannten Tensid-Gruppen, sowie jede Mischung von Tensiden eingesetzt werden.

Der Anteil des Cotensids, bezogen auf das Tensid, liegt bevorzugt im Bereich von 0,01 bis 99,99 %, insbesondere zwischen 1 und 50 %, besonders bevorzugt zwischen 5 und 25 %.

Geeignet sind, je nach Einsatzgebiet der erfindungsgemäßen Mischungen, beispielsweise alle klassischen Reiniger-Tenside, oder Lebensmittel-zugelassene Tenside, wie Tweens® oder Spans®. Von den Tensidklassen her sind geeignet nicht-ionische, anionische, kationische, amphotere Tenside; insbesondere auch Polymertenside, Peptidtenside, Silikontenside, aminosäurebasierte Tenside, Zuckertenside, fettbasierte Tenside, Geminitenside, Aminoxide, Amidoaminoxide, Alkylbetaine, Ethercarboxylate, Ampho-Acetate, Alkylsulfate oder Sulfosuccinate.

Geeignete anionische Tenside sind beispielsweise Fettalkoholsulfate von Fettalkoholen mit 8 bis 22, vorzugsweise 10 bis 18 Kohlenstoffatomen, zum Beispiel C₉- bis C₁₁-Alkoholsulfate, C₁₂- bis C₁₃-Alkoholsulfate, Cetylsulfat, Myristylsulfat, Palmitylsulfat, Stearylsulfat und Talgfettalkoholsulfat.

Weitere geeignete anionische Tenside sind sulfatierte ethoxylierte C₈- bis C₂₂-Alkohole (Alkylethersulfate) bzw. deren lösliche Salze. Verbindungen dieser Art werden beispielsweise dadurch hergestellt, das man zunächst einen Cg- bis C₂₂-, vorzugsweise einen C₁₀- bis C₁₈-Alkohol, zum Beispiel einen Fettalkohol, alkoxyliert und das Alkoxylierungsprodukt anschließend sulfatiert. Für die Alkoxylierung verwendet man vorzugsweise Ethylenoxid, wobei man pro Mol Fettalkohol 2 bis 50, vorzugsweise 3 bis 20 mol Ethylenoxid einsetzt. Die Alkoxylierung der Alkohole kann jedoch auch mit Propylenoxid allein und gegebenenfalls Butylenoxid durchgeführt werden. Geeignet sind außerdem solche alkoxylierte C₈- bis C₂₂-Alkohole, die Ethylenoxid und Propylenoxid oder Ethylenoxid und Butylenoxid enthalten. Die alkoxylierten C₈- oder bis C₂₂-Alkohole können die Ethylenoxid-, Propylenoxid- und butylenoxideinheiten in Form von Blöcken oder in statistischer Verteilung enthalten.

Geeignet sind auch Alkansulfonate, wie C₈- bis C₂₄-, vorzugsweise C₁₀- bis C₁₈-Alkansulfonate sowie Seifen, wie Na oder K-Salze von C₈- bis C₂₄-Carbonsäuren.

Weitere geeignete anionische Tenside sind N-Acylsarkosinate mit aliphatischen gesättigten oder ungesättigten C₈- bis C₂₅-Acylresten, vorzugsweise C₁₀- bis C₂₀-Acylresten, zum Beispiel N-Oleoylsarkosinat.

Weiterhin können die erfindungsgemäßen Mischungen C₁₀- bis C₁₃-lineare und/oder -leicht verzweigte Alkylbenzolsulfonate (LAS) enthalten.

Die anionischen Tenside werden der Mischung vorzugsweise in Form von Salzen zugegeben. Geeignete Kationen in diesen Salzen sind Alkalimetallsalze wie Natrium, Kalium und Lithium und Ammoniumsalze wie zum Beispiel Hydroxyethylammonium-, Di(hydroxyethyl)ammonium- und Tri(hydroxyethyl)ammoniumsalze.

Geeignete nichtionische Tenside sind insbesondere:
- alkoxylierte C₈- bis C₂₂-Alkohole wie Fettalkoholalkoxylate oder Oxoalkoholalkoxylate. Diese können mit Ethylenoxid, Propylenoxid und/oder Butylenoxid alkoxyliert sein. Als Tenside einsetzbar sind hierbei sämtliche alkoxylierten Alkohole, die mindestens zwei Moleküle eines der vorstehend genannten Alkylenoxide addiert enthalten. Hierbei kommen Blockpolymerisate von Ethylenoxid, Propylenoxid und/oder Butylenoxid in Betracht oder Anlagerungsprodukte, die die genannten Alkylenoxide in statistischer Verteilung enthalten. Die nichtionischen Tenside enthalten pro Mol Alkohol im allgemeinen 2 bis 50, vorzugsweise 3 bis 20 Mol mindestens eines Alkylenoxids. Vorzugsweise enthalten diese als Alkylenoxid Ethylenoxid. Die Alkohole haben vorzugsweise 10 bis 18 Kohlenstoffatome. Je nach Art des bei der Herstellung verwendeten Alkoxylierungskatalysators weisen die Alkoxylate eine breite oder enge Alkylenoxid-Homologenverteilung auf;
- Alkylphenolalkoxylate wie Alkylphenolethoxylate mit C₆- bis C₁₄-Alkylketten und 5 bis 30 Alkylenoxideinheiten;
- Alkylpolyglucoside mit 8 bis 22, vorzugsweise 10 bis 18 Kohlenstoffatomen in der Alkylkette und im allgemeinen 1 bis 20, vorzugsweise 1,1 bis 5 Glucosideinheiten Sorbitanalkanoate, auch alkoxyliert;
- N-Alkylglucamide, Fettsäurealkoxylate, Fettsäureaminalkoxylate, Fettsäureamidalkoxylate, Fettsäurealkanolamidalkoxylate, alkoxyliert, Blockcopolymere aus Ethylenoxid, Propylenoxid und/oder Butylenoxid, Polyisobuten-Ethoxylate, Polyisobuten-Maleinsäureanhydrid-Derivate, Monoglyceride, auch alkoxyliert sowie Bisglyceride.

Besonders geeignete nichtionsiche Tenside sind Alkylalkoxylate oder Gemische von Alkylalkoxylaten, wie sie beispielsweise in DE-A 102 43 363, DE-A 102 43 361, DE-A 102 43 360, DE-A 102 43 365, DE-A 102 43 366, DE-A 102 43 362 oder in DE-A 43 25 237 beschrieben sind. Hierbei handelt es sich um Alkoxylierungsprodukte, die durch Umsetzung von Alkanolen mit Alkylenoxiden in Gegenwart von Alkoxylierungskatalysatoren erhalten wurden oder um Gemische von Alkoxylierungsprodukten. Besonders geeignete Starteralkohole sind die sogenannten Guerbet-Alkohole, insbesondere Ehtylhexanol, Propylheptanol und Butyloktanol. Besonders bevorzugt ist Propylheptanol. Bevorzugte Alkylenoxide sind Propylenoxid und Ethylenoxid, wobei Alkylalkoxylate mit direkter Anbindung eines bevorzugt kurzen Poylypropylenoxidblocks an den Starteralkohol, wie sie beispielsweise in DE-A 102 43 365 beschrieben sind, insbesondere aufgrund ihres geringen Restalkoholgehalts und ihrer guten biologischen Abbaubarkeit bevorzugt sind.

Als Alkoxylierungskatalysatoren können Basen eingesetzt werden, beispielsweise Alkalihydroxide oder Alkalialkoholate jedoch auch Lewis-Säuren, beispielsweise BF₃, SbCl₅, SnCl₄ x 2H₂O, BF₃ x H₃BO₄, oder BF₃-Dietherat. Besonders geeignete Alkoxylierungskatalysatoren sind Doppelhydroxid-Tone wie Hydrotalkit, die insbesondere mit Additiven modifiziert sein können, wie in DE-A 43 25 237 beschrieben.

Je nach Wahl des Alkoxylierungskatalysators resultieren jeweils spezifische Eigenschaften der Alkoxylate, insbesondere bezüglich der Verteilung des Alkoxylierungsgrades. So werden bei Verwendung der letztgenannten Doppelhydroxid-Tone Alkoxylierungsprodukte mit einer engen Molekulargewichtsverteilung bzw. Homologenverteilung erhalten, die für den Einsatz in den erfindungsgemäßen Mischungen mit Cotensiden besonders geeignet sind.

Die vorstehend beschriebenen vorteilhaften Eigenschaften, insbesondere bezüglich des Alkoxylierungsgrades, werden auch durch Einsatz von Doppelmetallcyanid (DMC)-Verbindungen erreicht, wie sie beispielsweise in DE-A 102 43 361 als Alkoxylierungskatalysatoren beschrieben sind.

Gegenstand der Erfindung ist auch die Verwendung einer Mischung, enthaltend ein Tensid und ein vorstehend beschriebenes Cotensid zum Stabilisieren von Emulsionen, insbesondere von Mikroemulsionen. Stabilisieren bedeutet im vorliegenden Zusammenhang, dass durch Zusatz von Cotensiden die Effizienz von Tensiden gesteigert wird, das heißt die Solubilisierung eines definierten Öl/Wasser-Gemisches unter definierten Bedingungen mi einer geringeren Menge an Tensid ermöglicht wird.

Besonders bevorzugt eignen sich die vorstehend beschriebenen Cotenside zur Stabilisierung von Mikroemulsionen, das heißt zur Verschiebung des so genannten X-Punktes, der die niedrigste Konzentration an Tensid bei gegebener Temperatur darstellt, ab der der thermodynamische Zustand der Mikroemulsion, das heißt der in makroskopischer Betrachtung einphasige Zustand, eintritt.

Die erfindungsgemäßen Mischungen sind grundsätzlich in allen Bereichen einsetzbar, in denen Emulsionen eine Rolle spielen, beispielsweise in den in DE-A 101 18 480 aufgeführten Anwendungsbereichen für Mischungen umfassend ein Tensid und ein AB-Blockcopolymerisat als Additiv (Cotensid), die daneben Zusatzstoffe enthalten, deren Effizienz durch das Tensid/Additivsystem gesteigert werden kann: Beispielsweise als Pflanzenstärkungs-, -wuchs- oder Pflanzenschutzmittel, Produkte mit mikrobioziden Wirkstoffen, Produkte mit positiv oder negativ wirkenden Mikroorganismen, insbesondere mit einem Gehalt an Enzymen, Reinigugns- und/oder Pflegemittel für den Haushalt und für gewerbliche Zwecke, Desinfektionsmittel, Haar-, Körperpflege- oder Reinigungsmittel, Fahrzeugreinigungs-, -pflege- und/oder -konservierungsmittel, Textilbehandlungsmittel, Leder- und/oder Pelzpflegemittel, als Farben, Lacke, Arzneimittel, Bauhilfsstoffe, Zahnpasten oder Mundspülmittel.

Synergetische Effekte, wie sie in DE-A 101 18 480 für das System Tensid/AB-Blockcopolymerisat in Verbindung mit zusätzlichen Bioziden, Mikroorganismen und/oder beliebigen anderen Wirkstoffen beschrieben sind, werden entsprechend für Systeme enthaltend die erfindungsgemäßen Mischungen umfassend ein Tensid und ein Cotensid sowie entsprechende Zusatzstoffe, insbesondere Biozide, Mikroorganismen und/oder beliebige andere Wirkstoffe, erreicht.

Gegenstand der Erfindung ist auch eine Mikroemulsion, enthaltend ein Tensid und ein Cotensid, die wie vorstehend definiert sind.

Die erfindungsgemäßen Mischungen oder Mikroemulsionen sind optimal geeignet für die Aufnahme und Abgabe von Hydrophoben, insbesondere die Verwendung als Waschmittel, Emulgatoren, Schaumregulierer, Netzmittel für harte Oberflächen oder als Reaktionsmedium für organische, anorganische, bioorganische oder photochemische Reaktionen.

Bevorzugt ist die Verwendung in Waschmitteln, Tensidformulierungen, zur Reinigung harter Oberflächen, Feuchthaltemitteln, kosmetischen, pharmazeutischen und Pflanzenschutzformulierungen, Lacken, Beschichtungsmitteln, Klebstoffen, Lederentfettungsmitteln, Formulierungen für die Textilindustrie, Faserverarbeitung, Metallverarbeitung, Lebensmittelindustrie, Wasserbehandlung, Papierindustrie, Fermentation, Mineralverarbeitung, Brandschutz oder in Emulsionspolymerisationen.

Gegenstand der Erfindung sind auch Wasch-, Reinigungs-, Netz-, Beschichtungs-, Klebe-, Lederentfettungs-, Feuchthalte- oder Textilbehandlungsmittel oder pharmazeutische, Pflanzenschutz- oder kosmetische Formulierung, insbesondere Sonnenschutz-Hautpflege- oder Haarstylingmittel, Duschgele, Shampoos, Badezusätze oder Duftöle, enthaltend neben üblichen Inhaltsstoffen eine Mischung umfassend ein Tensid und ein wie vorstehend beschriebenes Cotensid oder eine Mikroemulsion, umfassend ein Tensid und ein Cotensid.

Die Erfindung wird im Folgenden anhand einer Zeichnung sowie von Anwendungsbeispielen näher erläutert.

In der Zeichnung zeigen im Einzelnen:

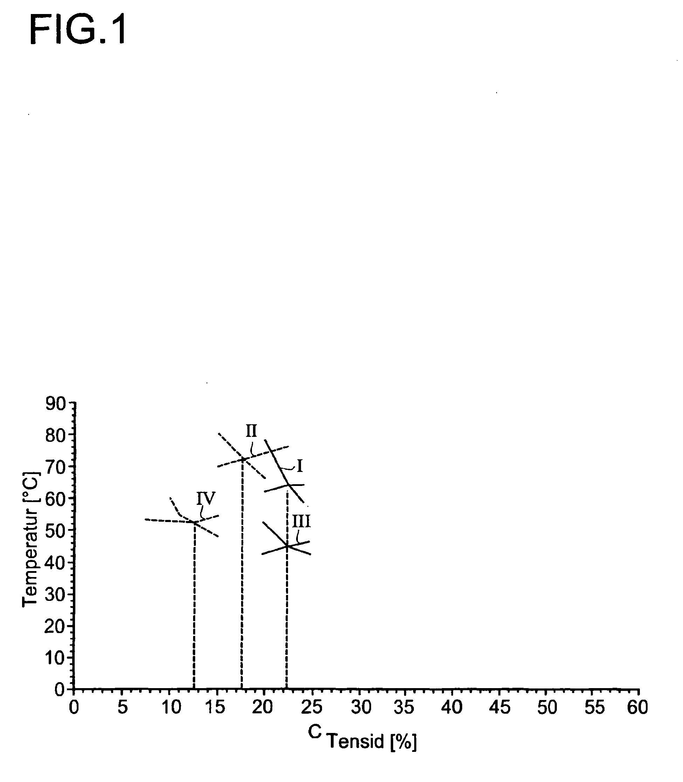

- Figur 1: den Einfluss des Tensids (hergestellt in DMC-Katalyse oder nicht) auf die Verschiebung des X-Punktes und

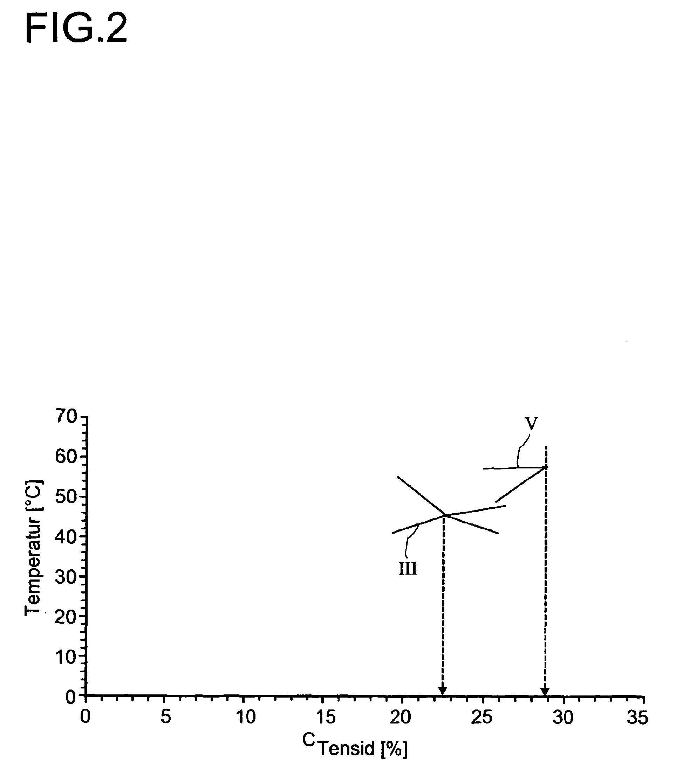

- Figur 2: den Einfluss von nicht-funktionalisiertem Polyisobuten auf die Verschiebung des X-Punktes derselben Mikroemulsion.

Der X-Punkt bezeichnet dabei in bekannter Weise die Mindestkonzentration an Tensid bei gegebener Temperatur, ab der für ein gegebenes Referenzsystem (vorliegend Wasser/n-Dekan) und ein gegebenes Tensid (vorliegend Lutensol® ON50 der BASF AG) die Wasser und die n-Dekan-Phase vollständig mischbar sind und eine thermodynamisch stabile Mikroemulsion ohne Exzess-Wasser- oder Ölphase ausbilden.

### Anwendungsbeispiel 1:

### Einfluss des Tensids auf den X-Punkt

In der anliegenden Figur ist auf der Abszisse die Konzentration des Tensids (Lutensol^{®} ON50 der BASF AG), in der Figur mit c_{Tensid}, in Gew.-% berechnet, und auf der Ordinate die Temperatur in °C dargestellt. Ausschnitte aus den jeweiligen Phasendiagrammen ("Fisch-Phasendiagramme") sind für das genannte Referenzsystem Wasser/-n-Dekan 1 : 1 unter I und III zum Vergleich, d.h. ohne Zusatz eines Cotensids und unter II und IV als erfindungsgemäße Anwendungsbeispiele mit Zusatz von jeweils 10 Gew.-% PIB-OH1000, ethoxyliert mit 82 Ethylenoxideinheiten, hergestellt. Das Diagramm I wurde mit dem Tensid Lutensol^{®} ON50 der BASF AG erhalten, das Diagramm III mit einem Lutensol^{®} ON50, das in DMC-Katalyse erhalten wurde. Die Figur zeigt, dass die Verschiebung des X-Punktes für ein nicht in DMC-Katalyse hergestelltes Tensid (Verschiebung von I nach II) bei Zusatz derselben Menge an Cotensid lediglich 5% beträgt, gegenüber einer Verschiebung (von III nach IV) um 7,5% bei Verwendung eines Tensids mit enger Homologenverteilung, das in DMC-Katalyse erhalten wurde.

### Anwendungsbeispiel 2 (Vergleich):

### Einfluss von nicht-funktionalisiertem Polyisobuten auf den X-Punkt

Das "Fisch-Phasendiagramm III" entspricht dem vorstehend unter Anwendungsbeispiel 1 beschriebenen Phasendiagramm für Lutensol® ON50 der BASF AG.

Zum Vergleich wurde nicht-funktionalisiertes Polyisobuten zugesetzt, das heißt Polyisobuten mit nicht-umgesetzten Doppelbindungen. Das erhaltene Phasendiagramm V zeigt, dass sich dadurch der X-Punkt zu höheren Tensidkonzentrationen verschiebt, das heißt, dass sich die Effizienz des Tensids verschlechtert. Das Anwendungsbeispiel zeigt somit, dass der Einsatz von Polyisobutenen, die dem Gegenstand der vorliegenden Patentanmeldung nicht entsprechen, zur Verschlechterung der Effizienz von Tensiden führt. Erfindungsgemäß wird dagegen von Polyisobuten-Blöcken ausgegangen, die zumindest 50 Mol-% terminal angeordnete Doppelbindungen aufweisen, das heißt von so genanntem reaktivem Polyisobuten.

## Patentansprüche

1. Mischung, umfassend ein Tensid und ein Cotensid, wobei das Cotensid ein amphiphiles Polymer, aufweisend eine oder mehrere hydrophobe Untereinheiten (A) und eine oder mehrere hydrophile Untereinheiten (B) ist, **dadurch gekennzeichnet, dass** eine oder mehrere hydrophobe Untereinheiten (A) auf der Basis eines Polyisobuten-Blockes erhalten durch kationische Polymerisation mit einer Lewis-Säure gebildet sind, dessen Polyisobuten-Makromoleküle zu mindestens 50 Mol-% terminal angeordnete Doppelbindungen aufweisen.

2. Mischung nach Anspruch 1, **dadurch gekennzeichnet, dass** jede hydrophobe Untereinheit (A) auf der Basis eines Polyisobuten-Blockes gebildet ist, dessen Polyisobuten-Makromoleküle zu mindestens 50 Mol-% terminal angeordnete Doppelbindungen aufweisen.

3. Mischung nach Anspruch 1 oder 2, **dadurch gekennzeichnet, dass** der Polyisobuten-Block aus Polyisobuten-Makromolekülen gebildet ist, wovon wenigstens 60 Mol-%, bevorzugt wenigstens 80 Mol-%, bezogen auf die Gesamtzahl der Polyisobuten-Makromoleküle, terminal angeordnete Doppelbindungen aufweisen.

4. Mischung nach einem der Ansprüche 1 bis 3, **dadurch gekennzeichnet, dass** der Polyisobuten-Block ein zahlenmittleres Molekulargewicht Mₙ im Bereich von 200 bis 20000 Dalton, bevorzugt im Bereich von 200 bis 5000 Dalton, aufweist.

5. Mischung nach einem der Ansprüche 1 bis 4, **dadurch gekennzeichnet, dass** der Polyisobuten-Block einen Polydispersitätsindex (PDI) im Bereich von 1,05 bis 10, bevorzugt im Bereich von 1,05 bis 5, besonders bevorzugt im Bereich von 1,05 bis 2, aufweist.

6. Mischung nach einem der Ansprüche 1 bis 5, **dadurch gekennzeichnet, dass** eine oder mehrere hydrophile Untereinheiten (B) aus sich wiederholenden Ethylenoxid- oder Ethylenoxid-/Propylenoxid-Einheiten gebildet sind, bevorzugt mit einem Anteil von 0-50 % Propylenoxid, besonders bevorzugt mit einem Anteil von 5-20 % Propylenoxideinheiten.

7. Mischung nach einem der Ansprüche 1 bis 5, **dadurch gekennzeichnet, dass** eine oder mehrere hydrophile Untereinheiten (B) aus Monomereinheiten, ausgewählt aus der nachfolgenden Gruppe: (Meth)acrylsäure, auch teilweise oder vollständig neutralisiert, (Meth)acrylate, Vinylacetat, Vinylalkohol, Vinylpyrrolidon, Allylalkohol, Styrol sowie hydrophile Derivate der vorstehend aufgeführten Monomereinheiten oder aus Mischungen hiervon gebildet sind.

8. Mischung nach einem der Ansprüche 1 bis 7, **dadurch gekennzeichnet, dass** man den Polyisobuten-Block unter Einführung von polaren Gruppen funktionalisiert und den funktionalisierten Polyisobutenblock anschließend gegebenenfalls weiter modifiziert.

9. Mischung nach Anspruch 8, **dadurch gekennzeichnet, dass** man die Funktionalisierung des Polyisobuten-Blocks durch eine Umsetzung, die aus der nachfolgenden Aufzählung ausgewählt ist, durchführt:
i) Umsetzung mit aromatischen Hydroxyverbindungen in Gegenwart eines Alkylierungskatalysators unter Erhalt von mit Polyisobutenen alkylierten aromatischen Hydroxyverbindungen,
ii) Umsetzung des Polyisobuten-Blocks mit eienr Peroxi-Verbindung unter Erhalt eines epoxidierten Polyisobutens,
iii) Umsetzung des Polyisobuten-Blocks mit einem Alken, das eine elektronenarme Doppelbindung aufweist (Enophil), in einer En-Reaktion,
iv) Umsetzung des Polyisobuten-Blocks mit Kohlenmonoxid und Wasserstoff in Gegenwart eines Hydroformylierungskatalysators unter Erhalt eines hydroformylierten Polyisobutens,
v) Umsetzung des Polyisobuten-Blocks mit einem Phosphorhalogenid oder einem Phosphoroxychlorid unter Erhalt eines mit Phosphongruppen funktionalisiertem Polyisobutens,
vi) Umsetzung des Polyisobuten-Blocks mit einem Boran und anschließender oxidativer Spaltung unter Erhalt eines hydroxylierten Polyisobutens,
vii) Umsetzung des Polyisobuten-Blocks mit einer SO₃-Quelle, bevorzugt Acetylsulfat unter Erhalt eines Polyisobutens mit terminalen Sulfonsäuregruppen,
viii) Umsetzung des Polyisobuten-Blocks mit Stickoxiden und anschließende Hydrierung unter Erhalt eines Polyisobutens mit terminalen Aminogruppen.

10. Mischung nach einem der Ansprüche 1 bis 9, **dadurch gekennzeichnet, dass** das Cotensid eine AₚB_{q} Struktur, wobei p und q unabhängig voneinander jeweils einen ganzen Wert zwischen 1 und 8 annehmen oder eine Kammstruktur aus A und B, aufweist.

11. Mischung nach einem der Ansprüche 1 bis 10, **dadurch gekennzeichnet, dass** man als Tensid ein Tensid mit enger Homologenverteilung, insbesondere ein unter DMC-Katalyse erhaltenes Tensid, einsetzt.

12. Verwendung einer Mischung nach einem der Ansprüche 1 bis 11 zum Stabilisieren von Emulsionen, insbesondere Mikroemulsionen.

13. Mikroemulsion, enthaltend ein Tensid und ein Cotensid, **dadurch gekennzeichnet, dass** man ein Cotensid entsprechend der Definition in einem der Ansprüche 1 bis 10 und/oder ein Tensid mit enger Homologenverteilung, insbesondere ein unter DMC-Katalyse erhaltenes Tensid einsetzt.

14. Verwendung einer Mischung nach einem der Ansprüche 1 bis 11 oder einer Mikroemulsion nach Anspruch 13 als Waschmittel, Emulgator, Schaumregulierer, Netzmittel für harte Oberflächen oder als Reaktionsmedium für organische, anorganische, bioorganische oder photochemische Reaktionen.

15. Verwendung nach Anspruch 14 in Waschmitteln, Tensidformulierungen zur Reinigung harter Oberflächen, Feuchthaltemitteln, kosmetischen, pharmazeutischen und Pflanzenschutzformulierungen, Lacken, Beschichtungsmitteln, Klebstoffen, Lederentfettungsmitteln, Formulierungen für die Textilindustrie, Faserverarbeitung, Metallverarbeitung, Lebensmittelindustrie, Wasserbehandlung, Papierindustrie, Fermentation, Mineralverarbeitung, Brandschutz oder in Emulsionspolymerisationen.

16. Wasch-, Reinigungs-, Netz-, Beschichtungs-, Klebe-, Lederentfettungs-, Feuchthalte- oder Textilbehandlungsmittel oder pharmazeutische, Pflanzenschutz- oder kosmetische Formulierung, insbesondere Sonnenschutz-Hautpflege- oder Haarstylingmittel, Duschgele, Shampoos, Badezusätze oder Duftöle, enthaltend neben üblichen Inhaltsstoffen eine Mischung nach einem der Ansprüche 1 bis 11 oder eine Mikroemulsion nach Anspruch 13.

## Claims

1. A mixture comprising a surfactant and a cosurfactant, the cosurfactant being an amphiphilic polymer having one or more hydrophobic subunits (A) and one or more hydrophilic subunits (B), wherein one or more hydrophobic subunits (A) have been formed on the basis of a polyisobutene block, obtained by cationic polymerization with a Lewis acid, whose polyisobutene macromolecules have terminal double bonds to an extent of at least 50 mol%.

2. The mixture according to claim 1, wherein every hydrophobic subunit (A) has been formed on the basis of a polyisobutene block whose polyisobutene macromolecules have terminal double bonds to an extent of at least 50 mol%.

3. The mixture according to claim 1 or 2, wherein the polyisobutene block has been formed from polyisobutene macromolecules of which at least 60 mol%, preferably at least 80 mol%, based on the total number of polyisobutene macromolecules, have terminal double bonds.

4. The mixture according to any one of claims 1 to 3, wherein the polyisobutene block has a number-average molecular weight Mₙ in the range from 200 to 20 000 daltons, preferably in the range from 200 to 5000 daltons.

5. The mixture according to any of claims 1 to 4, wherein the polyisobutene block has a polydispersity index (PDI) in the range from 1.05 to 10, preferably in the range from 1.05 to 5, more preferably in the range from 1.05 to 2.

6. The mixture according to any of claims 1 to 5, wherein one or more hydrophilic subunits (B) have been formed from repeat ethylene oxide units or ethylene oxide/propylene oxide units, preferably with a fraction of 0-50% propylene oxide, more preferably with a fraction of 5-20% propylene oxide units.

7. The mixture according to any of claims 1 to 5, wherein one or more hydrophilic subunits (B) have been formed from monomer units selected from the following group: (meth)acrylic acid, including partly or fully neutralized (meth)acrylic acid, (meth)acrylates, vinyl acetate, vinyl alcohol, vinylpyrrolidone, allyl alcohol, styrene and hydrophilic derivatives of the monomer units listed above, or from mixtures thereof.

8. The mixture according to any of claims 1 to 7, wherein the polyisobutene block is functionalized with introduction of polar groups and the functionalized polyisobutene block is then modified further if appropriate.

9. The mixture according to claim 8, wherein the functionalization of the polyisobutene block is carried out by a reaction which is selected from the following list:
i) reaction with aromatic hydroxyl compounds in the presence of an alkylation catalyst to obtain aromatic hydroxyl compounds alkylated with polyisobutenes,
ii) reaction of the polyisobutene block with a peroxy compound to obtain an epoxidized polyisobutene,
iii)reaction of the polyisobutene block with an alkene which has an electron-poor double bond (enophile) in an ene reaction,
iv) reaction of the polyisobutene block with carbon monoxide and hydrogen in the presence of a hydroformylation catalyst to obtain a hydroformylated polyisobutene,
v) reaction of the polyisobutene block with a phosphorus halide or a phosphorus oxychloride to obtain a polyisobutene functionalized with phosphone groups,
vi) reaction of the polyisobutene block with a borane and subsequent oxidative cleavage to obtain a hydroxylated polyisobutene,
vii)reaction of the polyisobutene block with an SO₃ source, preferably acetyl sulfate, to obtain a polyisobutene with terminal sulfonic acid groups,
viii) reaction of the polyisobutene block with nitrogen oxides and subsequent hydrogenation to obtain a polyisobutene with terminal amino groups.

10. The mixture according to any of claims 1 to 9, wherein the cosurfactant has an AₚB_{q} structure where p and q are each independently an integer from 1 to 8, or a comb structure composed of A and B.

11. The mixture according to any of claims 1 to 10, wherein the surfactant used is a surfactant with narrow homolog distribution, in particular a surfactant obtained under DMC catalysis.

12. The use of a mixture according to any of claims 1 to 11 for stabilizing emulsions, in particular microemulsions.

13. A microemulsion comprising a surfactant and a cosurfactant, wherein a surfactant as defined in any of claims 1 to 10 and/or a surfactant with narrow homolog distribution, especially a surfactant obtained under DMC catalysis, is used.

14. The use of a mixture according to any of claims 1 to 11 or of a microemulsion according to claim 13 as detergent, emulsifier, foam regulator, wetting agent for hard surfaces or as reaction medium for organic, inorganic, bioorganic or photochemical reactions.

15. The use according to claim 14 in detergents, surfactant formulations for the cleaning of hard surfaces, humectants, cosmetic, pharmaceutical and crop protection formulations, paints, coatings, adhesives, leather degreasing compositions, formulations for the textile industry, fiber processing, metal processing, food industry, water treatment, paper industry, fermentation, mineral processing, fire protection or in emulsion polymerizations.

16. A detergent, cleaner, wetting agent, coating, adhesive, leather degreasing composition, humectant or textile treatment composition or a pharmaceutical, crop protection or cosmetic formulation, in particular sunscreen, skincare or hair styling composition, shower gel, shampoo, bath additive or scent oil, comprising, as well as customary ingredients, a mixture according to any of claims 1 to 11 or a microemulsion according to claim 13.

## Revendications

1. Mélange, comprenant un tensioactif et un co-tensioactif, dans lequel le co-tensioactif est un polymère amphiphile présentant une ou plusieurs sous-unités hydrophobes (A) et une ou plusieurs sous-unités hydrophiles (B), **caractérisé en ce qu'**une ou plusieurs sous-unités hydrophobes (A) sont formées sur la base d'un bloc de polyisobutène obtenu par polymérisation cationique avec un acide de Lewis, dont les macromolécules de polyisobutène présentent au moins 50 % en moles de doubles liaisons disposées de manière terminale.

2. Mélange selon la revendication 1, **caractérisé en ce que** chaque sous-unité hydrophobe (A) est formée sur la base d'un bloc de polyisobutène, dont les macromolécules de polyisobutène présentent au moins 50 % en moles de doubles liaisons disposées de manière terminale.

3. Mélange selon la revendication 1 ou 2, **caractérisé en ce que** le bloc de polyisobutène est formé à partir de macromolécules de polyisobutène, dont au moins 60 % en moles, de manière préférée au moins 80 % en moles, par rapport au nombre total des macromolécules de polyisobutène, présentent des doubles liaisons disposées de manière terminale.

4. Mélange selon l'une quelconque des revendications 1 à 3, **caractérisé en ce que** le bloc de polyisobutène présente un poids moléculaire Mₙ moyen numérique dans la plage de 200 à 20 000 Daltons, de manière préférée dans la plage de 200 à 5000 Daltons.

5. Mélange selon l'une quelconque des revendications 1 à 4, **caractérisé en ce que** le bloc de polyisobutène présente un indice de polydispersité (PDI) dans la plage de 1,05 à 10, de manière préférée dans la plage de 1,05 à 5, de manière particulièrement préférée dans la plage de 1,05 à 2.

6. Mélange selon l'une quelconque des revendications 1 à 5, **caractérisé en ce qu'**une ou plusieurs sous-unités hydrophiles (B) sont formées à partir d'unités oxyde d'éthylène ou d'unités oxyde d'éthylène/unités oxyde de propylène se répétant, de manière préférée avec une part de 0 à 50 % d'unités oxyde de propylène, de manière particulièrement préférée avec une part de 5 à 20 % d'unités d'oxyde de propylène.

7. Mélange selon l'une quelconque des revendications 1 à 5, **caractérisé en ce qu'**une ou plusieurs sous-unités hydrophiles (B) sont formées à partir d'unités monomères, sélectionnées parmi le groupe suivant : acide (méth)acrylique, également partiellement ou complètement neutralisé, (méth)acrylates, acétate de vinyle, alcool vinylique, vinylpyrrolidone, alcool allylique, styrène ainsi que des dérivés hydrophiles des unités monomères présentées ci-dessus, ou à partir de mélanges de ceux-ci.

8. Mélange selon l'une quelconque des revendications 1 à 7, **caractérisé en ce que** le bloc de polyisobutène est fonctionnalisé avec introduction de groupes polaires et le bloc de polyisobutène fonctionnalisé est ensuite éventuellement encore modifié.

9. Mélange selon la revendication 8, **caractérisé en ce que** la fonctionnalisation du bloc de polyisobutène est mise en oeuvre grâce à une réaction qui est sélectionnée parmi la liste suivante:
i) réaction avec des composés hydroxy aromatiques en présence d'un catalyseur d'alkylation avec obtention de composés hydroxy aromatiques alkylés par des polyisobutènes,
ii) réaction du bloc de polyisobutène avec un composé peroxy, avec obtention d'un polyisobutène époxydé,
iii)réaction du bloc de polyisobutène avec un alcène qui présente une double liaison déficiente en électrons (énophile), dans une réaction ène,
iv) réaction du bloc de polyisobutène avec du monoxyde de carbone et de l'hydrogène en présence d'un catalyseur d'hydroformylation avec obtention d'un polyisobutène hydroformylé,
v) réaction du bloc de polyisobutène avec un halogénure de phosphore ou un oxychlorure de phosphore avec obtention d'un polyisobutène fonctionnalisé par des groupes phosphone,
vi) réaction du bloc de polyisobutène avec un borane et scission oxydative consécutive avec obtention d'un polyisobutène hydroxylé,
vii)réaction du bloc de polyisobutène avec une source de SO₃, de manière préférée de l'acétylsulfate avec obtention d'un polyisobutène ayant des groupes acide sulfonique terminaux,
viii) réaction du bloc de polyisobutène avec des oxydes nitriques et hydrogénation consécutive avec obtention d'un polyisobutène ayant des groupes amino terminaux.

10. Mélange selon l'une quelconque des revendications 1 à 9, **caractérisé en ce que** le co-tensioactif présente une structure AₚB_{q}, dans laquelle p et q, indépendamment l'un de l'autre, admettent respectivement une valeur entière comprise entre 1 et 8, ou une structure en peigne à partir de A et B.

11. Mélange selon l'une quelconque des revendications 1 à 10, **caractérisé en ce qu'**un tensioactif ayant une répartition étroite d'homologues, en particulier un tensioactif obtenu par catalyse DMC, est utilisé en tant que tensioactif.

12. Utilisation d'un mélange selon l'une quelconque des revendications 1 à 11 pour la stabilisation d'émulsions, en particulier de microémulsions.

13. Microémulsion, contenant un tensioactif et un co-tensioactif, **caractérisé en ce qu'**un co-tensioactif correspondant à la définition de l'une quelconque des revendications 1 à 10 et/ou un tensioactif ayant une répartition étroite d'homologues, en particulier un tensioactif obtenu par catalyse DMC, est utilisé.

14. Utilisation d'un mélange selon l'une quelconque des revendications 1 à 11 ou d'une microémulsion selon la revendication 13 en tant que détergent, émulsifiant, régulateur de mousse, réticulant pour des surfaces dures ou en tant que milieu réactionnel pour des réactions organiques, minérales, bio-organiques ou photochimiques.

15. Utilisation selon la revendication 14 dans des détergents, des formulations tensioactives pour le nettoyage de surfaces dures, des agents mouillants, des formulations cosmétiques, pharmaceutiques et phytosanitaires, des peintures, des produits de revêtement, des colles, des produits pour le dégraissage du cuir, des formulations pour l'industrie textile, le traitement des fibres, le traitement des métaux, l'industrie alimentaire, le traitement de l'eau, l'industrie papetière, la fermentation, le traitement des minerais, la protection contre l'incendie ou dans des polymérisations en émulsion.

16. Détergents, produits de nettoyage, agents de mouillage, produits de revêtement, colles, produits pour le dégraissage du cuir, agents mouillants ou agents de traitement pour le textile ou formulations pharmaceutiques, phytosanitaires ou cosmétiques, en particulier soins dermatologiques de protection contre le soleil ou produits de modelage des cheveux, gels douche, shampooings, additifs pour le bain et huiles parfumées, contenant, en plus des ingrédients usuels, un mélange selon l'une quelconque des revendications 1 à 11 ou une microémulsion selon la revendication 13.
